# EUROPÄISCHE PATENTSCHRIFT

(11) **EP 2 236 122 B1**
(45) Veröffentlichungstag und Bekanntmachung des Hinweises auf die Patenterteilung: **30.08.2017**
(21) Anmeldenummer: 10003232.5
(22) Anmeldetag: 26.03.2010
(51) Int. Cl.: A61K 6/083, A61K 6/00

(54) **Kunststoffmodifizierter Glasionomerzement**
Polymer-modified glass ionomer cement
Ciment en verre ionomère modifié par polymère

(30) Priorität: 02.04.2009 DE 102009016025
(43) Veröffentlichungstag der Anmeldung: 06.10.2010
(73) Patentinhaber: VOCO GmbH, 27472 Cuxhaven (DE)
(72) Erfinder: Christmann, Lars, Dr., 27476 Cuxhaven (DE); Plaumann, Manfred, Thomas, 27472 Cuxhaven (DE)

(56) Entgegenhaltungen:
- US-A1- 2004 065 228
- US-A1- 2005 252 413
- US-A1- 2006 160 919
- US-A1- 2008 314 286

## Beschreibung

Die vorliegende Erfindung betrifft kunststoffmodifizierte Glasionomerzemente zur Restauration von Zähnen, insbesondere zur Füllung von Milchzähnen, zur Unterfüllung, zum Stumpfaufbau, zur Befestigung dentaler und kieferorthopädischer Vorrichtungen, zur Versiegelung, für den Einsatz in der Endodontie, zur Herstellung künstlicher Zahnkronen sowie zur Alterszahnheilkunde. Die Erfindung betrifft ferner Verfahren zur Herstellung der Zemente, Verpackungseinheiten dieser Massen sowie die aus den Massen hergestellten Polymerisate.

Kunststoffmodifizierte dentale Glasionomerzemente sind aus dem Stand der Technik bekannt und werden durch Beigabe von Kunststoffkomponenten zu konventionellen Glasionomerzementen dargestellt.

Ursprüngliches Ziel bei der Entwicklung kunststoffmodifizierter Glasionomerzemente war die Verbesserung der physikalischen Eigenschaften und eine Vereinfachung der Handhabung herkömmlicher Glasionomerzemente. Konventionelle Glasionomerzemente weisen gegenüber Kompositmaterialien niedrigere Abrasions- und Zugfestigkeiten, ein schlechteres ästhetisches Erscheinungsbild sowie eine hohe Anfälligkeit für Verarbeitungsfehler auf. Insbesondere sind Glasionomerzemente während ihrer Anwendung sehr empfindlich gegenüber Feuchtigkeit: Gelangt der Zement in einer frühen Stufe seiner Aushärtung in Kontakt mit Feuchtigkeit, wie beispielsweise mit Speichel, dann wird der Fortgang der Abbindung gestört, so daß die physikalischen Eigenschaften des gehärteten Zements verschlechtert sind. Insbesondere können die abgebundenen Produkte dann eine erhöhte Löslichkeit im feuchten Milieu des Mundes aufweisen. Um diese Nachteile auszugleichen, wurden Mischformen entwickelt, bei denen die klassischen Inhaltsstoffe der bekannten Glasionomerzemente um Elemente dentaler Kunststoffe ergänzt wurden.

Die so entstandenen kunststoffmodifizierten Glasionomerzemente besitzen eine deutlich reduzierte Löslichkeit und bessere mechanische Eigenschaften als herkömmliche Glasionomerzemente. Ein wesentlicher Aspekt bei dieser Verbesserung ist die rasche Aushärtung und die hydrophobere Natur des Dentalmaterials.

Bei der Aushärtung der kunststoffmodifizierten Glasionomerzemente reagiert zunächst die Monomermischung in einer schnellen Vernetzung durch eine radikalische Reaktion. Auf diesem Weg wird die Löslichkeit der verbleibenden Zementbestandteile erheblich gemindert, da diese in die Polymermatrix eingeschlossen werden und das Material erhält eine erste "Härtestufe". Durch die beginnende Verfestigung des Materials wird das Glasionomersystem feuchtigkeitsunempfindlicher und kann dann, kinetisch langsamer, ungestört weiter durch die Säure-Base Reaktion des Glasionomers abbinden. Hierbei reagiert eine Polycarbonsäure mit einem reaktiven Glaspulver. Der Begriff "reaktives Glaspulver" bedeutet, daß das basische Glas "reaktiv" gegenüber einer Säurefunktion ist. Die Säure- Base Reaktion erfordert immer die Anwesenheit von Wasser.

Kunststoffmodifizierte Glasionomerzemente sind zweikomponentig formuliert und werden entweder als Paste/Paste Systeme, in der Form Pulver/Flüssigkeit, Pulver/Paste oder Flüssigkeit/Paste angewendet.

Die Aushärtereaktion wird in dem Moment eingeleitet, in dem die Komponenten miteinander vermischt werden. Die GIZ Reaktion erfolgt formal als Neutralisation der Säuregruppen des wasserlöslichen Polymers mit dem basischen Silikatglas. Das Glas kann beispielsweise ein Strontiumaluminiumfluorsilikatglas sein.

Für den Fall, daß die radikalische Polymerisation kunststoffmodifizierter Glasionomerzemente chemisch erfolgt, beginnen der GIZ-Abbindemechanismus und die redoxinitiierte Polymerisation zeitlich parallel, wobei die radikalische Reaktion kinetisch sehr viel schneller abläuft.

Für den Fall, daß die radikalische Reaktion kunststoffmodifizierter Glasionomerzemente durch Licht induziert wird, beginnt die Polymerisation zeitlich in dem Moment, wenn eine geeignete Lichtquelle die Monomermischung aktiviert. Auch hierbei läuft die radikalische Vernetzung sehr viel schneller als die GIZ-Abbindung ab.

Für den Fall, daß die radikalische Polymerisation kunststoffmodifizierter Glasionomerzemente sowohl durch Licht als auch chemisch erfogt, weist das System drei unterschiedliche Härtungsmechanismen mit drei unterschiedlichen kinetischen Abläufen auf.

EP 0 323 120 B1 beschreibt durch Licht härtbare Glasionomerzementzusammensetzungen vom Typ Pulver/Flüssigkeit. Durch die Abgabe von Fluoridionen weisen diese Materialien auch kariostatische Eigenschaften auf, die herkömmliche Restaurationsmaterialien auf Kunststoffbasis nicht aufweisen. Als reaktive Gläser werden bevorzugt ionenfreisetzende Fluoraluminiumsilikatgläser und Fluoraluminiumboratgläser eingesetzt. Die hergestellten Glasfritten werden zerkleinert und das Glaspulver durch ein Sieb mit einer Maschenweite von 44 µm abgetrennt.

Die WO 88/05651 beschreibt einen kunststoffmodifizierten radioopaken Glasionomerzement als Unterfüllungsmaterial vom Typ Paste/Paste, der entweder chemisch oder mittels Licht gehärtet wird. Wie in der EP 0 323 120 B1 wurde auch dieses System mit Blick auf die kariostatische Wirkung durch Abgabe von Fluoridionen formuliert. Die maximale Teilchengröße der reaktiven Glaspartikel liegt bei ca. 30 µm.

In der US 5,154,762 wird ein kunststoffmodifizierter Glasionomerzement offenbart, der 3-fach in einer Glasionomerzementreaktion, in einer chemisch induzierten Vernetzung und in einer Photopolymerisation aushärtet. Das reaktive Glaspulver wurde nach Siebung durch ein Sieb mit einer Weite von 74 µm erhalten.

Die EP 0 219 058 B2 betrifft kunststoffmodifizierte Glasionomerzemente vom Typ Pulver/Flüssigkeit. Die hier beschriebenen Systeme können sowohl chemisch als auch mittels Licht gehärtet werden. Bezüglich der für die Abbindung der Zemente wichtigen reaktiven Pulverkomponente verweist die Druckschrift u.a. auf die folgenden fünf Dokumente:
In der DE 20 61 513 wird angegeben, daß der Feinheitsgrad des Pulvers so gewählt werden sollte, daß beim Vermischen mit dem Anrührwasser eine glatte Zementpaste entsteht, die in einer klinisch günstigen Zeit härtet. Die Korngröße des Pulvers sollte weniger als 100 µm und noch besser weniger als 40 µm betragen.

In der DE 27 51 069 C2 wird angegeben, daß die Vermahlung des Pulvers vorzugsweise in der Weise erfolgen sollte, daß das Material zu Teilchen mit einem Durchmesser von weniger als 42 µm vermahlen wird.

In der DE 27 50 326 C3 wird angegeben, daß das Glas beispielsweise in einer Kugelmühle gemahlen wird bis eine Teilchengröße von etwa 100 µm, vorzugsweise von etwa 50 µm oder weniger erreicht ist. Um gepulverte Glasteilchen mit einer gleichmäßig kleinen Korngröße auszulesen, werden die Teilchen, die zu groß sind, durch Sieben entfernt. Das Pulver sollte so fein sein, daß man damit eine glatte, klinisch verträgliche, pastenartige Zementmasse bilden kann. Das gepulverte Glas hat vorzugsweise einen solchen Feinheitsgrad, daß es durch ein Sieb mit einer lichten Maschenweite von 104 µm, insbesondere 53 µm hindurchgeht.

In der US 4,250,277 wird angegeben, daß das reaktive Glas Teilchen in Größen umfassen kann, die kleiner als 250 µm, vorzugsweise kleiner als 75 µm sind.

In der EP 0 023 013 B1 wird angegeben, daß die erfindungsgemäßen Glaspulver eine durchschnittliche Korngröße von wenigstens 0,5 µm, bevorzugt wenigstens 1 µm und besonders bevorzugt mindestens 3 µm aufweisen. Für Dentalzwecke beträgt die die durchschnittliche Korngröße 1 - 20 µm, bevorzugt 3 - 15 µm und besonders bevorzugt 3 - 10 µm. Die Teilchen haben eine maximale Korngröße von 150 µm, vorzugsweise 100 µm, besonders 60 µm. Für die Verwendung als dentaler Befestigungszement beträgt die maximale Teilchengröße 25 µm, vorzugsweise 20 µm.

EP 0 329 268 A2 offenbart durch Licht härtbare, kunststoffmodifizierte Glasionomerzemente vom Typ Pulver/Flüssigkeit. Duch die Lichthärtung wird dem System eine frühe mechanische Festigkeit mitgegeben. Durch seine zeitlich versetzte Abbindung ist der Zement in der Lage, seine physikalischen Eigenschaften weiter zu verbessern. Zusätztlich entfaltet er die für Glasionomerzemente typische Haftung an Zahnsubstanz. Das reaktive Glaspulver wird auf Größen im Bereich von ca. 0,5 µm bis 10 µm gemahlen und in den Zement eingesetzt.

Die DE 199 14 975 A1 betrifft u.a. auch kunststoffmodifizierte Glasionomerzemente vom Typ Pulver/Flüssigkeit, die oberflächenmodifizierte Komponenten aufweisen. Die polymerisierbaren Verbindungen sind in den Polyelekrolytzementen ganz bevorzugt zu 7 - 15 Gew.-% enthalten. Die Systeme können chemisch, thermisch oder mittels Licht vernetzt werden.

Die DE 10 2006 014 772 A1 offenbart kunststoffmodifizierte Glasionomerzemente vom Typ Pulver/Flüssigkeit, die chemisch aushärten. Das reaktive Glaspulver weist eine Teilchengröße von 0,2 µm bis 20 µm auf.

In der DE 298 25 053 U1 werden chemisch (und optional photochemisch) härtende, kunststoffmodifizierte Glasionomerzemente vom Typ Pulver/Flüssigkeit vorgeschlagen, die die Beschichtung des Pulvers mit Initiator und Aktivator vorsehen. Die ionenfreisetzenden Gläser werden mit einer Teilchengröße von vorzugsweise 0,1 bis 50 µm, insbesondere 1 bis 20 µm eingesetzt.

Die EP 1 269 968 B1 beschreibt chemisch härtende, kunststoffmodifizierte Glasionomerzemente vom Typ Paste/Paste und vom Typ Pulver/Flüssigkeit. Im Dokument wird festgestellt, daß die Oberfläche des ausgehärteten Zements rau erscheint und der Zement schlecht verarbeitbar ist, wenn grobe reaktive Gläser mit einer mittleren Größe von > 20 µm eingesetzt werden. Andererseits sollen die mechanischen Eigenschaften schlecht werden, wenn das reaktive Glas eine mittlere Größe von < 0,02 µm aufweist. Die Oberfläche des reaktiven Glaspulvers wird dann so groß, daß nur noch geringe Mengen in die organische Harzmatrix eingearbeitet werden können. Das reaktive Glaspulver sollte somit bestenfalls eine mittlere Teilchengröße von 0,02 µm bis 20 µm aufweisen.

In der DE 39 41 629 C2 wird über einen kunststoffmodifizierten Glasionomerzement vom Typ Pulver/Flüssigkeit, Paste/Flüssigkeit oder Paste/Paste berichtet, der chemisch oder photochemisch härtet und grenzflächenaktive Mittel enthält. Die mittlere Teilchengröße des reaktiven Glaspulvers beträgt hier zwischen 0,02 µm und 10 µm. Bei einer mittleren Teilchengröße von von mehr als 10 µm soll keine Oberflächenglätte des gehärteten Zements durch Polieren erzielt werden. Dies soll ein schlechtes Gefühl im Mund vermitteln. Ferner soll die Aushärtungsreaktion mit der Polyacrylsäure in ungünstiger Weise verzögert werden. Wird ein feines Pulver von < 0,02 µm verwendet, sollen die physikalischen Eigenschaften reduziert werden.

Die US 7,173,074 B2 beschreibt chemisch aushärtbare, zweikomponentige, kunststoffmodifizierte Glasionomerzemente vom Typ Pulver/Flüssigkeit und Paste/Paste. Das reaktive Glaspulver weist eine mittlere Teilchengröße von wenigstens ca. 0,2 µm und vorzugsweise wenigstens ca. 1 µm auf. Die mittlere Teilchengröße ist nicht größer als ca. 10 µm und vorzugsweise nicht größer als ca. 5 µm. In den Beispielen wurden die Gläser (FAS (Fluoraluminiumsilikat) I bis VI) durch Durchsieben des Pulvers durch Siebe mit Weiten von 60 und 74 µm erhalten. Unterschiede zwischen den Gläsern betreffen auch die Modifizierung ihrer Oberflächen. In den Beispielen wurden auch binäre Mischungen der Glasvarianten eingesetzt.

Die US 2006/0030637 A1 schlägt chemisch härtende, zweikomponentige, kunststoffmodifizierte Glasionomerzemente vor, denen auch Photoinitiatoren zugegeben werden können. Die Zemente können vom Typ Pulver/Flüssigkeit, Paste/Flüssigkeit oder Paste/Paste sein. Durch das Vorhandensein von Salzen sollen sie bessere Lagerstabilitäten aufweisen. Die mittlere Größe des reaktiven Glases ist nicht größer als ca. 10 µm, vorzugsweise nicht größer als ca. 5 µm.

Die DE 10 2006 019 092 A1 betrifft chemisch härtende, zweikomponentige, kunststoffmodifizierte Glasionomerzemente vom Typ Paste/Paste. In dem Dokument wird angegeben, daß das reaktive Glaspulver einen durchschnittlichen Teilchendurchmesser von 0,01 bis 30 µm, bevorzugter von 0,01 bis 10 µm haben sollte, falls das Material zum Füllen von Kavitäten oder für eine Kernkonstruktion verwendet wird, also dort, wo eine hohe mechanische Festigkeit des Werkstoffs erforderlich ist. Wird das Material zur Befestigung eingesetzt, also dort, wo geringe Filmdicken erforderlich sind, soll der durchschnittliche Teilchendurchmesser des reaktiven Glaspulvers zwischen 0,01 und 10 µm, bevorzugter zwischen 0,01 und 5 µm liegen. Auch in dieser Schrift wird angegeben, daß das reaktive Glaspulver nicht kleiner als 0,01 µm sein sollte, da dann, aufgrund der großen Oberfläche der reaktiven Glasphase, die Menge des Glases im Material begrenzt ist und somit wichtige Eigenschaften des Materials, wie beispielsweise die mechanische Festigkeit, erniedrigt ist. Sollte das reaktive Glaspulver einen durchschnittlichen Teilchendurchmesser von > 30 µm aufweisen, dann wäre die ausgehärtete Materialoberfläche, beipielsweise einer Zahnfüllung, nach dem Polieren rau und hätte wenig Glanz. Sollte das reaktive Glaspulver eine durchschnittliche Größe von 10 µm übersteigen und das Material zur Zementierung verwendet werden, dann könnte die erforderliche Filmdicke zu dick werden und Restaurationen wie Kronen und Brücken würden nicht haften.

In der US 2005/0252414 A1 findet sich der Vorschlag, die Ästhetik eines kunststoffmodifizierten Glasionomerzements, der chemisch und/oder photochemisch härtbar ist, durch Beigabe eines Nanofüllstoffs zu verbessern. Durch die Gegenwart des Nanofüllstoffs soll der Brechungsindex der flüssigen Phase erhöht werden und so zu einem besseren Angleich der Brechungsindices der Matrixphase mit der festen Phase führen. Dieser Angleich soll dazu führen, daß das ausgehärtete Material eine verbesserte optische Transluzenz aufweist. In dem Dokument wird weiter angegeben, daß die mittlere Größe des reaktiven Glaspulvers nicht mehr als 12 µm, üblicherweise nicht mehr als 10 µm und am üblichsten nicht mehr als 5 µm beträgt. In den Beispielen werden drei unterschiedliche reaktive Pulver eingesetzt. Ein reaktives Glaspulver weist eine durchschnittliche Teilchengröße von 1 µm auf, ein anderes reaktives Glaspulver hat eine Partikelgößenverteilung, die durch den d₉₀ Wert charakterisiert ist, der 9 µm beträgt und ein drittes reaktives Glas besitzt eine Teilchengrößenverteilung mit einem d₉₀ Wert von 27 µm. Der d₉₀ Wert bezieht sich auf die Volumenverteilung und besagt, daß 90% des Volumens der gemessenen Teilchen unterhalb des bestimmten Wertes liegen. In den Ausführungsbeispielen wird jeweils nur eine reaktive Glaspulverfraktion verwendet.

Die DE 602 09 714 T2 beschreibt ebenfalls kunststoffmodifizierte Glasionomerzemente. Bevorzugte durchschnittliche Partikeldurchmesser für die eingesetzten reaktiven Glaspulver sind 0,2 bis 15 µm und insbesondere 1 bis 10 µm. In den Beispielen wurden das Glaspulver FAS (Fluoraluminiumsilikat) I durch Durchsieben des Pulvers durch ein 60 µm Sieb und das Glaspulver FAS II durch Durchsieben des Pulvers durch ein 74 µm Sieb erhalten (siehe hierzu auch oben die US 7,173,074 B2). Die Glaspulver FAS III und FAS IV wurden analog erhalten, waren jedoch gegenüber den Varianten FAS I und FAS II unterschiedlich oberflächenbehandelt. Es wurde eine weitere Variante FAS V angegeben, ein Gemisch aus FAS II und IV, die jedoch nicht beispielhaft eingesetzt wurde.

Die DE 194 26 224 B4 offenbart kunststoffmodifizierte Glasionomerzemente vom Typ Paste/Paste und Pulver/Flüssigkeit, die sowohl chemisch als auch photochemisch härtbar sind. Die Systeme sollen stark verbesserte physikalische Eigenschaften wie Anfangshärte, Biegefestigkeit und Adhäsionsfestigkeit an Dentin im Vergleich zu konventionellen Glasionomerzementen aufweisen. Lichtgehärtete Zementzusammensetzungen sollen selektiv für Füllungszwecke und chemisch härtende Zusammensetzungen selektiv für Befestigungszwecke einsetzbar sein. Das reaktive Glaspulver soll vorzugsweise eine mittlere Teilchengröße im Bereich von 0,02 - 10 µm aufweisen. In den Beispielen wird das reaktive Glaspulver durch Durchsieben des Pulvers durch ein Sieb mit einer Weite von 74 µm erhalten.

Die DE 196 05 272 B4 beschreibt kunststoffmodifizierte Glasionomerzemente, die ein reaktives Glaspulver enthalten, das durch Durchsieben des Glases durch ein Sieb der Weite 74 µm erhalten wurde.

Die EP 0 627 907 offenbart kunststoffmodifizierte Glasionomerzemente, die ein reaktives Glaspulver enthalten, das durch Durchsieben des Glases durch ein Sieb der Weite 44 µm erhalten wurde.

Kunststoffmodifizierte Glasionomerzemente werden auch in der DE 697 04 612 T2 vorgeschlagen, in der bevorzugte Teilchengrößenverteilungen des reaktiven Glaspulvers im Bereich von 0,02 bis 50 µm, bevorzugter im Bereich zwischen 0,1 bis 10 µm und am bevorzugtesten im Bereich zwischen 1 bis 6 µm liegen.

Weiterhin sind kunststoffmodifizierte Glasionomerzemente in den Druckschriften DE 695 33 844 T2 sowie in DE 694 16 333 T2 beschrieben, die mittlere Teilchengrößen des reaktiven Glaspulvers von 5,5 µm angeben.

In US 5,965,632 werden kunststoffverstärkte Glasionomerzemente vom Typ Paste/Paste vorgestellt, deren reaktives Glaspulver eine mittlere Teilchengröße im Bereich zwischen 1 und 15 µm aufweist.

Die US 5,859,089 schützt dentale Kompositmaterialien, die chemisch und/oder photochemisch aushärten können. Die Materialien enthalten reaktive Glaspulver. Das reaktive Glaspulver weist eine mittlere Teilchengröße von 0,05 - 20 µm, eine bevorzugte mittlere Teilchengröße von 0,1 - 10 µm und eine am meisten bevorzugte mittlere Teilchengröße von 0,3 - 5 µm auf. Diese Systeme sind einkomponentig und wasserfrei.

Die DE 198 11 811 B4 betrifft Harzzusammensetzungen, die sowohl photochemisch als auch chemisch gehärtet werden können. In dieser Patentschrift wird in die Kunststoffmatrix bevorzugt feines, reaktives Glaspulver mit einem maximalen Teilchendurchmesser von weniger als 10 µm und einem mittleren Teilchendurchmesser von weniger als 5 µm, insbesondere ein reaktives Glaspulver mit einem mittleren Teilchendurchmesser von 0,05 bis 8 µm und ganz besonders mit einem mittleren Teilchendurchmesser von 0,1 bis 2,0 µm eingesetzt. Das dentale Material soll Fluoridionen abgeben. Die Systeme sind einkomponentig und wasserfrei.

Die US 6,126,922 schützt eine einkomponentige, wasserfreie Harzzusammensetzung, die ebenfalls ein reaktives Glaspulver enthält. Das reaktive Glaspulver wurde durch Sieben des Glases durch ein 60 µm weites Sieb erhalten.

Die US 2006/160919 A1 beschreibt kunststoffmodifizierte Glasionomerzemente enthaltend a.) wenigstens ein polymer einer alpha, beta-ungesättigten Carbonsäure, b.) eine basische Glaszusammensetzung, die in der Lage ist, mit genanntem Polymer zu reagieren, c.) wenigstens ein radikalisch polymerisierbares Monomer. d.) Wasser, e.) wenigstens einen Polymerisationsinitiator. Die Glaszusammensetzung kann als Mischung von zwei Fraktionen mit unterschiedlichen durchschnittlichen Partikelgrößen (multimodal) vorliegen; die durchschnittliche Partikelgröße ist bevorzugt < 40 µm.

US 2004/065228 A1 beschreibt kunststoffmodifizierte Glasionomerzemente enthaltend Glaspartikel mit d₅₀ < 5 µm und d₉₉ < 20 µm.

US 2005/252413 A1 beschreibt kunststoffmodifizierte Glasionomerzemente, die eine Mischung von reaktiven Glasfüllstoffen mit den unterschiedlichen durchschnittlichen Partikelgrößen von 1 und 3 µm enthalten.

US 2008/314286 A1 beschreibt kunststoffmodifizierte Glasionomerzemente, die eine Mischung aus lonomerpartikeln mit einer engen Größenverteilung und Partikelgrößen zwischen 0,5 und 50µm enthalten.

Die oben beschriebenen kunststoffmodifizierten Glasionomerzemente weisen gegenüber den klassischen Glasionomerzementen einige verbesserte Eigenschaften wie Druckhärte, Biegefestigkeit und Löslichkeit auf. Dennoch sind die hier erreichten Werte noch verbesserungswürdig. Es war somit Aufgabe der vorliegenden Erfindung Zusammensetzungen auf der Grundlage eines kunststoffmodifizierten Glasionomersystems bereitzustellen, die verbesserte mechanische Eigenschaften gegenüber analogen Materialien aus dem Stand der Technik aufweisen. Insbesondere sollte auch das Abrasionsverhalten der erfindungsgemäßen Systeme verbessert werden.

Die oben benannten Aufgaben werden durch kunststoffmodifizierte Glasionomerzementzusammensetzungen gemäß Patentanspruch 1 gelöst. Weitere erfindungsmäßige Ausgestaltungen ergeben sich aus den Unteransprüchen.

Insbesondere betrifft die vorliegende Erfindung kunststoffmodifizierte Glasionomerzemente enthaltend
a.) wenigstens ein Polymer einer alpha, beta - ungesättigten Carbonsäure
b.) eine basische Glaszusammensetzung, die in der Lage ist, mit wenigstens einem Polymer einer alpha, beta - ungesättigten Carbonsäure zu reagieren
c.) wenigstens ein radikalisch polymerisierbares Monomer
d.) Wasser
e.) wenigstens einen Polymerisationsinitiator
f.) optional herkömmliche Additive wie Farbmittel, Weinsäure, Kieselsäure, gegenüber dem Polymer einer alpha, beta - ungesättigten Carbonsäure inerte Füllstoffe, Stabilisatoren, Inhibitoren, Modifikatoren und bakterizid wirkende Substanzen,
dadurch gekennzeichnet, daß die basische Glaszusammensetzung eine Kombination aus einem ersten reaktiven Glaspulver mit einem d₅₀ Wert von 1,5 µm bis 6 µm und bevorzugt von 4 µm und einem d₉₉ Wert von 7 µm bis 20 µm, bevorzugt von 6 µm bis 15 µm und ganz bevorzugt von 10 µm und einem zweiten reaktiven Glaspulver mit einem d₅₀ Wert von 7 µm bis 20 µm, bevorzugt von 7,5 µm bis 15 µm und ganz bevorzugt von 8 µm und einem d₉₉ Wert von 10 µm bis 25 µm, bevorzugt von 15 µm bis 22 µm und ganz bevorzugt von 20 µm darstellt, wobei der Anteil an dem ersten reaktiven Glaspulver den Anteil an dem zweiten reaktiven Glaspulver übersteigt.

Der d₅₀ Wert bezieht sich auf die Volumenverteilung und besagt, daß 50% des Volumens der gemessenen Teilchen unter- und 50% des Volumens oberhalb des bestimmten Wertes liegen.

Der d₉₉ Wert bezieht sich ebenfalls auf die Volumenverteilung und besagt, daß 99% des Volumens der gemessenen Teilchen unter- und 1% des Volumens oberhalb des bestimmten Wertes liegen.

Es wurde überraschenderweise gefunden, daß die mechanischen Eigenschaften wie Biegefestigkeit, Druckhärte und Abrasion, die Haftkraft an der Zahnhartsubstanz sowie das Klebeverhalten an den Applikationsinstrumenten gegenüber herkömmlichen kunststoffmodifizierten Glasionomersystemen deutlich verbessert werden, wenn eine klar definierte Auswahl bezüglich der Zusammensetzung des reaktiven Glaspulvers im kunststoffmodifizierten Glasionomerzement getroffen wird. Eine solche Auswahl ist im Stand der Technik unbekannt.

Bei dentalen Kunststoffen gibt es die Klasse der Hybridkomposite, bei denen eine definierte Auswahl unterschiedlicher Füllstoffe eingesetzt wird, in der Regel herkömmliches (nicht reaktives) Glas mit einer Partikelgröße von 0,6 - 1,5 µm sowie noch kleinere Kieselsäurepartikel im Größenbereich von 0,01 - 0,05 µm.

Bei der Formulierung geeigneter Kunststoffmaterialien kommt der Partikelgröße eine besondere Bedeutung zu. Große Teilchendurchmesser ergeben Komposite mit schlechter Polierbarkeit und schlechten Abrasionseigenschaften, während feinteilige Füllstoffe das Material stark verdicken, so daß der Füllstoffeintrag reduziert ist. Bedingt durch den geringen Füllstoffgehalt weisen diese Systeme dann einen hohen Polymerisationsschrumpf sowie schlechte mechanische Eigenschaften auf.

Aus diesen Gründen gibt es innerhalb der Gruppe von Hybridkompositen wiederum Systeme, die eine gezielte Auswahl unterschiedlicher Teilchengrößen des eingesetzten Füllstoffs treffen. Nur beispielhaft seien erwähnt:
EP 0 971 678 B1, hier wird ein reines Kompositsystem geschützt, dessen Füllstoffkomponente eine erste Menge an Glasteilchen mit einer mittleren Teilchengröße von 1 bis 10 µm, eine zweite Menge an Glasteilchen mit einer mittleren Teilchengröße von 0,1 bis 1 µm und eine Menge an Füllstoffteilchen mit einer mittleren Teilchengröße von 0,01 bis 0,04 µm aufweist.

DE 27 05 220 C2, hier wurde ein Verfahren zur Herstellung eines transparenten Dentalwerkstoffs mit hoher Druckfestigkeit geschützt, in denen ein feinteiliger Füllstoff mit einer solchen Kornverteilung eingesetzt wird, daß 70 - 95 Gew.-% der Teilchen eine Korngröße von 0,7 bis 25 µm und 5 - 30 Gew.-% der Teilchen eine Korngröße von 0,2 bis 0,7 µm aufweisen. Teilchen mit einem größeren Durchmesser als 25 µm sind nicht erwünscht, da ihre Anwesenheit zu einer unerwünschten Rauhigkeit der Oberfläche des Dentalwerkstoffs führe. Teilchen mit einem geringeren Durchmesser als 0,2 µm können verwendet werden, jedoch nicht in größeren Mengen als etwa 5 Gew.-% des Füllstoffs. Als feinteiliger Füllstoff wird hier roher alpha-Quarz eingesetzt.

DE 101 08 261 A1, hier werden Kompositmaterialien beschrieben, die als Füllstoff eine Mischung aus einem organisch - anorganischen Kompositfüller vorzugsweise mit einer mittleren Partikelgröße von 5 bis 50 µm, Glaspulver mit einer maximalen Partikelgröße von 10 µm und einer mittleren Partikelgröße von 0,1 bis 5 µm und einem feinteiligen Füllstoff mit einer Partikelgröße von 0,01 bis 0,04 µm enthalten. Der Kompositfüller ist mit Glaspulver gefüllt, das eine maximale Teilchengröße von 10 µm und eine mittlere Teichengröße von 0,1 bis 5 µm aufweist. Die Dentalmaterialien sollen sich durch eine glatte Oberfläche, einen geringen Polymerisationsschrumpf und verbesserte physikalische Eigenschaften auszeichnen.

DE 10 2005 021 332 B4, hier werden Kompositmaterialien geschützt, die in der Füllstoffkomponente neben nicht agglomerierten Nanofüllern mit Partikelgrößen von 1 - 50 nm auch ein Füllstoffgemisch aus grob- und feinteilgen Dentalgläsern, die ein Größenverhältnis, bezogen auf die mittlere Teilchengröße von feinteilig zu grobteilig von 1:4 bis 1:30 aufweisen.

Diese Systeme sind jedoch für die Lösung der hier gestellten Aufgabe ohne Relevanz, da die eingesetzten Füllstoffe nicht reaktiver Natur sind und die zugrunde liegenden Systeme reine, auf Kunststoff basierende Zusammensetzungen darstellen. Selbst wenn in den Kunststoffsystemen reaktive Gläser eingesetzt würden (beispielsweise wie in DE 197 57 647 A1), wäre es auch dann für die hier vorliegende Aufgabe ohne Belang, da die basischen Glaskomponenten keine Umsetzung mit einem Reaktionspartner eingehen. Dieser Umstand einer Doppelfunktion der erfindungsgemäßen (reaktiven) Glaszusammensetzungen, die sowohl die Reaktivität gegenüber einem Reaktionspartner und dadurch die Reaktionskinetik der Systemhärtung als auch Füllstofffunktionen wie oben dargelegt (mechanische Eigenschaften, Verarbeitungseigenschaften, ästhetische Eigenschaften des resultierenden Formstoffs) umfaßt, wird mit dieser Erfindung erstmals aufgegriffen und überraschend gelöst.

Die Erfindung wird im Folgenden näher beschrieben.

Das Polymer (a) einer alpha, beta - ungesättigten Carbonsäure entsteht nach einer radikalischen Polymerisation beispielsweise einer wäßrigen, alpha- beta ungesättigten Acryl-, Methacryl-, Itacon-, Fumar-, Malein-, Chlormethacryl-, Cyanomethacryl-, Aconit-, Mesacon-, Glutacon-, Citraconsäure, etc.. Werden die organischen Mono- oder Dicarbonsäuren (bzw. deren Anhydride) einzeln polymerisiert, so erhält man Homopolymere. Wird die Polymerisation in Gegenwart von zwei oder mehreren Säuren durchgeführt, so gelangt man zu Copolymeren. Erfindungsgemäß kann ein einziges Polymer oder ein Gemisch unterschiedlicher Polymere eingesetzt werden.

Die ungesättigten carbonsäuregruppenhaltigen Monomere sind nicht besonders beschränkt und können verwendet werden unabhängig von der Anzahl von Carbonsäuregruppen im Molekül oder der Existenz einer Carbonsäureanhydridgruppe oder anderer Substituenten. Der Einsatz der Homo- oder Copolymere kann in Form ihrer wäßrigen Lösungen erfolgen. Bevorzugt werden Polymere der Acryl- oder Maleinsäure eingesetzt.

Ist das gewichtsmittlere Molekulargewicht dieser Polymere kleiner als 1.000 g/mol, so sind die mechanischen Eigenschaften der resultierenden dentalen Massen ungenügend, die Haftung des Materials an der Zahnsubstanz ist schlecht und der Patient verspürt einen schlechten Geschmack und vernimmt einen unangenehmen Geruch. Übersteigt das gewichtsmittlere Molekulargewicht des Polymers einen Wert von 150.000 g/mol, so steigt die Viskosität so stark, daß ein homogenes Vermischen der Komponenten unmöglich wird. Erfindungsgemäß liegt das mittlere Molekulargewicht des alpha, beta- ungesättigten Carbonsäurepolymers somit zwischen 1.000 und 150.000 g/mol und bevorzugt zwischen 40.000 und 100.000 g/mol.

Weiterhin ist es möglich, die carbonsäuregruppenhaltigen Polymere in fester Form einzusetzen. Hierzu werden die Polymerlösungen beispielsweise gefriergetrocknet oder anderen geeigneten Methoden unterworfen.

Die erfindungsgemäß eingesetzte basische Glaszusammensetzung (b), die in der Lage ist, mit einem Polymer einer alpha, beta - ungesättigten Carbonsäure zu reagieren, muß säurereaktive Elemente, beispielsweise Metallelemente aus den Gruppen I, II und III des Periodensystems enthalten. Diese können beispielsweise Natrium, Kalium, Calzium, Strontium, Aluminium, etc. sein. Das säurereaktive Glas kann ein oder mehrere Arten dieser Elemente aufweisen. Die säurereaktiven Elemente können in Form ihrer Oxide, Hydroxide, Sulfate, Nitrate, Phosphate, Carbonate, Silicate, Fluoride, Nitride, etc. verwendet werden. Bezüglich ihrer Mengenverhältnisse wird auf die Angaben in der DE 39 41 629, der DE 195 26 224 B4 und ähnlichen Dokumenten verwiesen, in denen auch gezeigt wird, wie die Eigenschaften des ausgehärteten Werkstoffs von Art und Menge der säurereaktiven Elemente und ihrer Gegenionen abhängen. Die Freisetzung der säurereaktiven Elemente aus dem Glas, also die Reaktivität des Glases gegenüber den Säurefunktionen, wird durch den gezielten Einbau von Fehlstellen im Gitter des Glases herbeigeführt. Dies kann beispielsweise durch die Variation der eingesetzten Mengen an dreiwertigen Kationen gegenüber Si ⁴⁺ erreicht werden. Eine andere Möglichkeit zur bewußten Generierung von Gitterfehlstellen im Glas stellt der Einsatz von Fluoridionen dar, da F⁻ gegenüber O ²⁻ den Aufbau eines regulären Gitters stört. Ansonsten ist der Gehalt der säurereaktiven Elemente nicht spezifisch beschränkt, so wie die chemische Zusammensetzung des Glases keinen besonderen Beschränkungen unterliegt.

Vorzugsweise wird ein Aluminiumfluorsilikatglas eingesetzt. Hauptkomponenten des Glases sind dann Al ³⁺, Si ⁴⁺, F⁻, O ²⁻, und Ca ²⁺ und/oder Sr ²⁺. Das Glas wird nach den bekannten Verfahren hergestellt. Beispielsweise verwendet man Siliziumdioxid, Aluminiumoxid, Aluminiumhydroxid, Aluminiumsilikat, Strontiumsilikat, Natriumfluorid, Aluminiumfluorid, Aluminiumphosphat, Natriumaluminiumfluorid und Strontiumphosphat. Das Rohmaterial wird gemischt und bei über 1000 °C geschmolzen, abgekühlt, gemahlen und nach Korngrößenfraktionen getrennt. Das Trennen der Fraktionen erfolgt beispielsweise durch Sieben oder Sichten. Die Korngrößen des eingesetzten reaktiven Glases unterliegen einer Beschränkung. Es wurde überraschenderweise gefunden, daß eine Kombination aus einer ersten reaktiven Glaspulverfraktion mit einem d₅₀ Wert von 1,5 µm bis 6 µm und bevorzugt von 4 µm und einem d₉₉ Wert von 7 µm bis 20 µm, bevorzugt von 7 µm bis 15 µm und ganz bevorzugt von 10 µm und einer zweiten reaktiven Glaspulverfraktion mit einem d₅₀ Wert von 7 µm bis 20 µm, bevorzugt von 7,5 µm bis 15 µm und ganz bevorzugt von 8 µm und einem d₉₉ Wert von 10 µm bis 25 µm, bevorzugt von 15 µm bis 22 µm und ganz bevorzugt von 20 µm, wobei der Anteil an der ersten reaktiven Glaspulverfraktion den Anteil an der zweiten reaktiven Glaspulverfraktion übersteigt, dem resultierenden Werkstoff ein ganz besonderes Eigenschaftsprofil hinsichtlich seiner Mechanik und seiner Verarbeitbarkeit verleiht.

Optional kann die Oberfläche des reaktiven Glaspulvers organisch modifiziert werden. Durch eine solche Behandlung kann die Kompatibilität des reaktiven Glaspulvers mit den anderen Komponenten der Zusammensetzung verbessert werden. Eine organische Oberflächenmodifizierung des reaktiven Glaspulvers ist jedoch nur dann angezeigt, solange die Freisetzung der reaktiven Elemente nicht behindert ist. Bevorzugt ist für den Fall einer Oberflächenmodifizierung die Durchführung einer Silanisierung. Hierbei wird die Oberfläche des reaktiven Glaspulvers beispielsweise mit einer organischen Silanverbindung, die über eine polymerisierbare, ethylenisch ungesättigte Doppelbindung verfügt, überzogen. Bevorzugt eingesetzt wird Vinyltrimethoxysilan oder insbesondere Methacryloxypropyltrialkoxysilan. Die Organosilanverbindungen können allein oder in Gemischen verwendet werden. Weitere Beispiele umfassen Silane, die keine ethylenisch ungesättigte Doppelbindung aufweisen, wie gamma-Aminopropyltrialkoxysilan, Propyltrialkoxysilan, etc..

Andere Oberflächenmodifizierungsmittel können Fettsäuren, organische Säuren, Tenside, anorganische Säuren, Polysiloxane, etc. sein.

Das radikalisch polymerisierbare Monomer (c) kann eine mindestens zwei ethylenische Gruppen aufweisende Substanz sein wie beispielsweise, ohne darauf beschränkt zu sein, eine in der Dentalchemie üblicherweise in Kompositmaterialien eingesetzte Kunststoffverbindung wie Ethylenglykoldimethacrylat, 1,6-Hexandioldimethacrylat, Triethylenglykoldimethacrylat, 1,12-Dodecandioldimethacrylat, ethoxyliertes Bisphenol-A-dimethacrylat, Polyethylenglykoldimethacrylat, 7,7,9-Trimethyl-4,13-dioxo-5,12-diazahexadecan-1,16-dioxydimethacrylat, Butandioldimethacrylat, Trimethylolpropantrimethacrylat, Pentaerythrittetramethacrylat, Dipentaerythrithexamethacrylat, Tetraethylenglykoldimethacrylat, Neopentylglykoldimethacrylat, etc.. In der Patentliteratur ist eine Vielzahl weiterer Verbindungen genannt (beispielsweise auch in der DE 39 41 629 A1), die allesamt Diester der Acryl- oder Methacrylsäure sind und sich zum Einsatz in einer erfindungsgemäßen Zusammensetzung eignen. Explizit verwendet werden können auch die entsprechenden Dimethacrylate bzw. Diacrylate des Dihydroxymethyltricyclo[5.2.1.0^{2,6}]decans, wie sie in den Druckschriften DE 1816823, DE 2419887, DE 2406557, DE 2931926, DE 3522005, DE 352206, DE 3703120DE 2005021332, DE 102005053775, DE 102006060983, DE 69935794 und DE 102007034457 beschrieben sind.

Das radikalisch polymerisierbare Monomer (c) kann auch eine mindestens eine ethylenische Doppelbindung aufweisende Hydroxylverbindung sein, beispielsweise, ohne darauf beschränkt zu sein, ebenfalls ein in der Dentalchemie üblicherweise eingesetzter Kunststoff wie 2- Hydroxyethylmethacrylat, 2- Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glyzerindimethacrylat, 2,3-bis[4-[3-methacryloyloxy-2-hydroxypropoxy]phenyl]propan, etc..

Die geeigneten Verbindungen können einzeln oder in Gemischen eingesetzt werden. Wasser (e) kann demineralisiert oder auch einfach als Leitungswasser verwendet werden. Der Polymerisationsinitiator (f) kann entweder ein Initiator zur chemischen Härtung der Zusammensetzung und/oder ein Initiator zur Lichthärtung der Zusammensetzung sein. Beispiele für einen chemischen Polymerisationsinitiator schließen Katalysatorsysteme vom Redoxtyp mit ein. Sie umfassen beispielsweise die Systeme "organische Peroxidverbindung/Amin", "organische Peroxidverbindung/Amin/Sulfinat", "organische Peroxidverbindung/Amin/Borat", etc.. Weitere Katalysatoren stellen organische Borverbindungen, Perborate, Permagnate und Persulfate dar. Sie können mit Sauerstoff oder Wasser reagieren. Es gibt ferner Sulfinate, Barbitursäuren und Boratverbindungen, die in Gegenwart von Wasser bzw. sauren Gruppen reagieren können.

Geeignete konkrete Beispiele dieser unterschiedlichen Systeme werden in der DE 10 2006 019 092 A1 sowie insbesondere in der DE 2006 014 772 beschrieben. Man kann ein System allein oder in Kombination mit einem anderen System verwenden. Bevorzugt werden Redoxsysteme eingesetzt, die aus den Elementen Barbituräure/-derivat, Peroxodisulfat/- diphosphat, Kupferverbindung, ionogen vorliegende Halogenverbindung bestehen. Besonders bevorzugt wird ein Redoxsystem verwendet, das 1-Benzyl-5-phenylbarbitursäure, Natriumperoxodisulfat, Kupferacetylacetonat und Benzyltributylammoniumchlorid enthält. Beispiele für einen Lichthärtungsinitiator schließen Katalysatoren ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc.. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2.

Beispiele für Beschleuniger, die zusammen mit den Sensibilisatoren eingesetzt werden, sind beispielsweise tertiäre Amine, sekundäre Amine, Barbitursäuren, Zinnverbindungen, Aldehyde und Schwefelverbindungen. Konkrete Substanzbeispiele der unterschiedlichen Klassen finden sich beispielsweise in der DE 10 2006 019 092 oder in der DE 39 41 629 C2. Weitere geeignete Initiatoren sowie Initiatorkombinationen sind in der DE 601 16 142 beschrieben.

Bevorzugt wird die Kombination eines alpha Diketons mit einem aromatischen tertiären Amin eingesetzt, insbesondere wird Campherchinon und Ethyl-p-N,N-dimethylaminobenzoat benutzt.

Besonders bevorzugt ist die weitere Kombination des Systems "alpha Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in einer erfindungsgemäßen Zusammensetzung wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha Diketon/Amin" als Photopolymerisationsinitiatorsystem in den erfindungsgemäßen Zusammensetzungen.

Die optional herkömmlichen Additive (g) umfassen als Farbmittel beispielsweise anorganische und organische Pigmente oder Farbstoffe. Beispiele hierfür werden in der DE 196 05 272 gegeben.

Die (+) Weinsäure stellt ein zusätzliches Mittel zur Kontrolle der Abbindereaktion zwischen dem reaktiven Glaspulver und dem Polymer einer alpha, beta - ungesättigten Carbonsäure dar, indem sie die Verarbeitungszeit verlängert, die Abbindungsgeschwindigkeit jedoch gleichzeitig beschleunigt.

Das reaktive Glaspulver kann in Kombination mit bekannten anorganischen Füllstoffen, die in breitem Umfang in dentalen Kompositwerkstoffen Verwendung finden, eingesetzt werden. Dies sind beispielsweise Barium-Aluminium-Borsilikatgläser unterschiedlicher Teilchengrößen, die nicht gegenüber einem Polymer einer alpha, beta - ungesättigten Carbonsäure reaktiv sind. Es können auch Quarz, Feldspat, ultrafeine Kieslsäure, Titandioxid oder Bariumsulfat der Glasmischung zugefügt werden. Der Einsatz nanoskaliger nicht agglomerierter Kieselsäure Partikel mit Teilchengrößen von 1 bis 200 nm, die aus dem Sol-Gel Verfahren stammen, führt zu einer noch besseren Raumerfüllung der Füllkörper, so daß die Eigenschaften der resultierenden Werkstoffe im Vergleich zu den Produkten aus dem Stand der Technik noch vorteilhafter werden. Nanoskalige, nicht aggregierte Sole sind in einer großen Bandbreite käuflich zu erwerben. Bevorzugt können auch Füllstoffe bestimmter Geometrie, wie sie beispielsweise durch Sprühtrocknung von Solen erhalten werden, verwendet werden. Beispiele hierfür sind aggregierte Kieselsäureteilchen in der Form von Tori, wie sie in der WO 00/25729 oder in der DE 102 53 481 beschrieben sind. Weitere Beispiele für geeignete Kieselsäuren sind Aerosile, wie Aerosil 200, Aerosil OX 50, Aerosil R 972, Aerosil R 974, Aerosil R 8200, Aerosil R 711, Aerosil DT 4, etc.. Zu den Aerosilen gehören auch Aluminiumoxid C sowie Titandioxid P 25. Darüberhinaus können organische und/oder organisch-anorganische Hybridfüllstoffe zum Einsatz kommen. Die gegenüber dem Polymer einer alpha, beta - ungesättigten Carbonsäure nicht reaktiven Feststoffe können ebenso wie das reaktive Glas organisch oberflächenmodifiziert sein.

Als Stabilisatoren können der erfindungsgemäßen Zusammensetzung beispielsweise Tenside und/oder Verdickungsmittel beigegeben werden.

Als Inhibitoren eigenen sich beispielsweise substituierte Phenole wie 2,6 Di-tert.-butyl-4-methylphenol (BHT) oder Hydrochinonmonomethylether (MEHQ). Diese Substanzen verhindern eine vorzeitige radikalische Polymerisation der Monomere, wenn diese beispielsweise dem Tageslicht ausgesetzt sind. Weitere mögliche geeignete Inhibitoren umfassen Phenothiazin und seine Derivate sowie stabile organische Radikale, die ebenfalls geeignete Inhibitoren darstellen. Beispiele für stabile organische Radikale sind das 2,2-Diphenyl-1-picrylhydrazyl-, das Galvonoxyl-, das Triphenylmethyl- und das 2,2,6,6-Tetramethylpiperidinyl-1-oxylradikal (TEMPO). Ebenfalls geeignet sind die Derivate von TEMPO. Beispiele für Derivate von TEMPO und Phenothiazin befinden sich beispielsweise in der EP 0 783 880 beschrieben. Die Inhibitoren können einzeln oder in Gemischen dem erfindungsgemäßen Zement zugesetzt werden.

Die erfindungsgemäßen Zusammensetzungen können in der Form Pulver/Flüssigkeit, Pulver/Paste, Paste/Flüssigkeit oder Paste/Paste vorliegen.

Für die Form Pulver/Flüssigkeit kann beispielsweise die wäßrige Lösung des Polymers einer alpha, beta - ungesättigten Carbonsäure mit den polymerisierbaren ungesättigten organischen Verbindungen, den Polymerisationsinitiatoren, den Inhibitoren, der Weinsäure und einer pyrogenen Kieselsäure zum Einstellen des rheologischen Verhaltens der flüssigen Phase versetzt werden. Die feste Phase kann dann beispielsweise die reaktiven Glasfraktionen, den zusätzlichen Füllstoff, den Coinitiator und möglicherweise einen Teil des Polymers der alpha, beta - ungesättigten Carbonsäure enthalten.

Für die Form Paste/Paste kann beispielsweise eine Paste, die das reaktive Glaspulver enthält, zusätzlich die gesamten (oder einen Teil der) polymerisierbaren ungesättigten organischen Verbindungen, Polymerisationsinitiatoren und Inhibitoren enthalten. Diese Paste wird dann mit einer anderen Paste kombiniert, die beispielsweise die wäßrige Lösung des Polymers einer alpha, beta - ungesättigten Carbonsäure, zusätzliche Füllstoffe, Weinsäure und einen Coinitiator enthält.

Die reaktiven Glaspulver lassen sich mit Hilfe von Modifikatoren sehr gut zu einer Paste verarbeiten. Als Modifikatoren können beispielsweise wasserlösliche Substanzen hohen Molekulargewichts wie Stärkederivate, Carboxyalkylzellulose, Polyvinylalkohol, Polyethylenglykol, Polyacrylamid, Polyvinylpyrrolidon, Xanthan Gum, Alginat, etc. eingesetzt werden. Eine solche Paste kann auch alle oder einen Teil der poylmerisierbaren Verbindungen sowie die Zusatzstoffe und Initiatoren aufnehmen.

Die Verarbeitung der erfindungsgemäßen Zusammensetzungen kann beispielsweise per Handanmischung erfolgen. Im Falle der Form Pulver/Flüssigkeit wird in der Regel ein sukzessiver Mischprozess ausgeführt, bei dem eine zuvor bestimmte Menge des Pulvers stufenweise mit einer zuvor bestimmten Menge der Flüssigkeit mit Hilfe eines Anmischinstruments miteinander vermengt wird. Analog wird man im Falle der Form Paste/Paste, Pulver/Paste oder Paste/Flüssigkeit vorgehen.

Alternativ kann die erfindungsgemäße Zusammensetzung im Falle der Form Pulver/Flüssigkeit auch mit Hilfe einer Misch- und Applikationskapsel angewendet werden. Bei einer solchen Kapsel handelt es sich um ein Instrument zur Aufnahme, zum Anmischen und Ausdrücken fließfähiger Werkstoffe. So kann die Flüssigkeit beispielsweise in einem in der Kapsel seitlich angebrachten Kissen aufbewahrt werden. Pulver und Flüssigkeit können so exakt vordosiert eingesetzt werden. Durch Aktivierung der Kapsel mit einem Aktivierungsinstrument kann beispielsweise das Flüssigkeitskissen durchstoßen werden, so daß die Kammer, die die feste Phase enthält, die Flüssigkeit aufnehmen kann. Die so aktivierte Kapsel kann dann mit einem automatischen Kapselmischgerät für eine festgelegte Mischzeit bei einer konstanten Mischfrequenz homogen vermischt werden. Nach Beendigung des Vermischens kann dann der Kapselinhalt mit Hilfe einer Applikationszange direkt auf den Zahn im Munde des Patienten ausgebracht werden. Beispiele für geeignete Misch- und Applikationskapseln finden sich in EP 1 029 513 B1, DE 39 20 537 A1, EP 1 219 262 A1, EP 1 226 790 A1, DE 100 07 581 A1, EP 0 157 121 A1, EP 0 627 200 B1, etc..

Selbstverständlich kann das Vermischen der erfindungsgemäßen Zusammensetzung im Falle der Form Paste/Paste auch maschinell und automatisch mit Hilfe eines entsprechenden Kartuschen- oder Spritzensystems mit statischem Mischrohr zur Direktapplikation erfolgen. Bei der Direktapplikation werden die Komponenten zunächst in entsprechend dimensionierte Doppelkammerspritzen abgefüllt. Die Applikation erfolgt dann durch Auspressen der Pasten aus der Doppelkammerspritze mit Hilfe eines Doppelstempels durch eine statische Mischkanüle auf den zu behandelnden Zahn im Mundinnenraum. Eine unterschiedliche Einfärbung der beiden ungemischten Ausgangsmassen erlaubt nach dem Durchgang der Pasten durch die Mischkanüle anhand des resultierenden Farbtons die Homogenität der Mischung zu überprüfen.

Das Mischungsverhältnis der beiden Komponenten kann zwischen 10:1 und 1:10 betragen und liegt bevorzugt zwischen 1:4 und 4:1.

Für unterschiedliche Indikationen werden im Allgemeinen unterschiedliche Mischungsverhältnisse eingestellt, um die unterschiedlichen Anforderungen erfüllen zu können. Für die erfindungsgemäßen Zusammensetzungen beträgt beispielsweise für die Form Pulver/Flüssigkeit das Mischungsverhältnis von Pulver zu Flüssigkeit für Befestigungen zwischen 1,4 und 2,0, für Unterfüllungen zwischen 2,1 und 2,5 und für Füllungen zwischen 2,1 und 3,3.

Aus diesen Unterschieden in den Zusammensetzungen geht hervor, daß die mechanischen Eigenschaften in Abhängigkeit von den Indikationen unterschiedlich sind. Ein Befestigungszement braucht keine niedrigen Abrasionswerte. Umgekehrt braucht ein Füllungsmaterial keine niedrige Filmdicke. Beide Systeme brauchen jedoch eine gute Haftung.

Die kunststoffmodifizierten Glasionomerzemente können direkt im Mund des Patienten am Zahn appliziert werden oder sie können auch außerhalb des Mundes beispielsweise an einer Prothese angewendet werden, wobei die Prothese anschließend im Mund verklebt wird.

Die Abbindung beginnt direkt nach dem Auftrag der Pasten auf den zu behandelnden Zahn. Im Falle eines chemisch härtenden Systems beginnt ebenfalls zeitlich parallel zur Abbindung die Vernetzung der Monomeren. Im Falle eines lichthärtenden Systems muß die Mischung durch das Anlegen einer geeigneten Strahlungsquelle zur Polymerisation aktiviert werden.

Im Folgenden wird die Erfindung anhand von Beispielen näher beschrieben.

### I. System Pulver/Flüssigkeit, lichthärtend, Hauptindikationen: Füllung; Unterfüllung, Stumpfaufbau, handangemischt, P/L (powder/liquid) = 2,5 : 1,0 (w/w)

**Flüssigkeit 1**

| | | | | | |
|---|---|---|---|---|---|
| Pos | Komponente | Anteil [%] | | Initiator 1: | D, L-Campherchinon |
| | | | | Initiator 2: | Phosphinoxid |
| 1 | Initiator 1 | 0,50 | | Inhibitor: | 3,5-Di-tert-butyl-4-hydroxytoluol |
| 2 | Initiator 2 | 0,50 | | Kieselsäure: | pyrogene Kieselsäure |
| 3 | Inhibitor | 0,05 | | Weinsäure: | (+) Weinsäure |
| 4 | Kieselsäure | 1,50 | | Monomer 1: | Hydroxyethylmethacrylsäureester (HEMA) |
| 5 | Weinsäure | 3,00 | | | |
| 6 | Monomer 1 | 31,95 | | Monomer 2: | Urethandimethacrylsäureester (UDMA) |
| 8 | Monomer 2 | 7,50 | | | |
| 9 | Polycarbonsäure | 20,90 | | Polycarbonsäure: | polymerisierte Acrylsäure |
| 10 | Wasser | 34,10 | | | |
| | Gesamt | 100,00 | | | |

**Flüssigkeit 2**

| | | | | | |
|---|---|---|---|---|---|
| Pos | Komponente | Anteil [%] | | Initiator 2: | Phosphinoxid |
| | | | | Initiator 1: | D, L-Campherchinon |
| 1 | Initiator 1 | 0,50 | | Inhibitor: | 3,5-Di-tert-butyl-4-hydroxytoluol |
| 2 | Initiator 2 | 0,50 | | Kieselsäure: | pyrogene Kieselsäure |
| 3 | Inhibitor | 0,05 | | Weinsäure: | (+) Weinsäure |
| 4 | Kieselsäure | 1,50 | | Monomer 1: | Glycerin-1,3-dimethacrylsäure-ester (GlyDMA)) |
| 5 | Weinsäure | 3,00 | | | |
| 6 | Monomer 1 | 10,50 | | Monomer 2: | Hydroxyethylmethacrylsäureester (HEMA) |
| 7 | Monomer 2 | 19,95 | | | |
| 8 | Monomer 3 | 9,00 | | Monomer 3: | Dihydroxymethyltricyclo-[5.2.1.0^{2,6}] decandimethacrylat |
| 9 | Polycarbonsäure | 20,90 | | | |
| 10 | Wasser | 34,10 | | Polycarbonsäure: | polymerisierte Acrylsäure |
| | Gesamt | 100,00 | | | |

**Pulver 1 - Pulver aus reaktivem Füllstoff mit Partikelgrößenverteilung d₅₀ = 8 µm, d₉₉ = 20 µm**

| Pos | Komponente | Anteil [%] |
|---|---|---|
| 1 | Coinitiator | 0,20 |
| 2 | Polycarbonsäure | 5,00 |
| 3 | reaktives Glas < 20 µm | 94,80 |
| | Gesamt | 100,00 |

| | | |
|---|---|---|
| Coinitiator: Dimethylaminobenzoesäureethylester Polycarbonsäure: polymerisierte Acrylsäure reaktives Glas: Fluoraluminosilikatglas | | |

**Pulver 2 - Pulver aus reaktivem Füllstoff mit Partikelgrößenverteilung d₅₀ = 4 µm, d₉₉ = 10 µm**

| Pos | Komponente | Anteil [%] |
|---|---|---|
| 1 | Coinitiator | 0,20 |
| 2 | Polycarbonsäure | 5,00 |
| 3 | reaktives Glas < 10µm | 94,80 |
| | Gesamt | 100,00 |

| | | |
|---|---|---|
| Coinitiator: Dimethylaminobenzoesäureethylester Polycarbonsäure: polymerisierte Acrylsäure reaktives Glas: Fluoraluminosilikatglas | | |

**Pulver 3 - Pulver aus Füllstoffkombination aus reaktivem Füllstoff mit Partikelgrößenverteilung d₅₀ = 4 µm, d₉₉ = 10 µm und reaktivem Füllstoff mit Partikelgrößenverteilung d₅₀ = 8 µm, d₉₉ = 20 µm**

| Pos | Komponente | Anteil [%] |
|---|---|---|
| 1 | Coinitiator | 0,20 |
| 2 | Polycarbonsäure | 5,00 |
| 3 | reaktives Glas <10µm | 75,00 |
| 4 | reaktives Glas <20µm | 19,80 |
| | Gesamt | 100,00 |

| | | |
|---|---|---|
| Coinitiator: Dimethylaminobenzoesäureethylester Polycarbonsäure: polymerisierte Acrylsäure reaktives Glas: Fluoraluminosilikatglas | | |

**Pulver 4 - Pulver aus Füllstoffkombination aus reaktivem Füllstoff mit Partikelgrößenverteilung d₅₀ = 4 µm, d₉₉ = 10 µm und reaktivem Füllstoff mit Partikelgrößenverteilung d₅₀ = 8 µm, d₉₉ = 20 µm**

| Pos | Komponente | Anteil %] |
|---|---|---|
| 1 | Coinitiator | 0,20 |
| 2 | Polycarbonsäure | 5,00 |
| 3 | reaktives Glas <10µm | 50,00 |
| 4 | reaktives Glas <20µm | 44,80 |
| | Gesamt | 100,00 |

| | | |
|---|---|---|
| Coinitiator: Dimethylaminobenzoesäureethylester Polycarbonsäure: polymerisierte Acrylsäure reaktives Glas: Fluoraluminosilikatglas | | |

**Pulver 5 - Pulver aus Füllstoffkombination aus reaktivem Füllstoff mit Partikelgrößenverteilung d₅₀ = 4 µm, d₉₉ = 10 µm und reaktivem Füllstoff mit Partikelgrößenverteilung d₅₀ = 8 µm, d₉₉ = 20 µm**

| Pos | Komponente | Anteil [%] |
|---|---|---|
| 1 | Coinitiator | 0,20 |
| 2 | Polycarbonsäure | 5,00 |
| 3 | reaktives Glas <10µm | 30,00 |
| 4 | reaktives Glas <20µm | 64,80 |
| | Gesamt | 100,00 |

| | | |
|---|---|---|
| Coinitiator: Dimethylaminobenzoesäureethylester Polycarbonsäure: polymerisierte Acrylsäure reaktives Glas: Fluoraluminosilikatglas | | |

**Pulver 6 - Pulver aus reaktivem Füllstoff mit Partikelgrößenverteilung d₅₀ = 8 µm, d₉₉ = 40 µm**

| Pos | Komponente | Anteil [%] |
|---|---|---|
| 1 | Coinitiator | 0,20 |
| 2 | Polycarbonsäure | 5,00 |
| 3 | reaktives Glas < 40µm | 94,80 |
| | Gesamt | 100,00 |

| | | |
|---|---|---|
| Coinitiator: Dimethylaminobenzoesäureethylester Polycarbonsäure: polymerisierte Acrylsäure reaktives Glas: Fluoraluminosilikatglas | | |

Die Anwendungsbeispiele 1 - 6 wurden mit der Flüssigkeit 1 und den Pulvern 1 - 6 und die Anwendungsbeispiele 7 - 12 wurden mit der Flüssigkeit 2 und den Pulvern 1 - 6 durchgeführt. Im Pulver 6 wird eine nach dem Stand der Technik übliche Partikelgrößenverteilung benutzt.

Die Untersuchungen wurden wie folgt durchgeführt:
DHT = Durchhärtetiefe: analog zu DIN EN ISO 4049:2008
DH = Druckhärte: analog zu DIN 9917-1:2004
BF = Biegefestigkeit: analog zu FDIS 9917-2:2008
Löslichkeit: analog ISO 7489:1986
Abrasion: Drei-Medien-Abrasionstest gem. ACTA-Methode, 100.000 Zyklen (ISO/TS 14569) Alle Anwendungsbeispiele wurden mit einem Massenverhältnis von Pulver zu Flüssigkeit (P/L) = 2,5 : 1,0 durchgeführt.

Die Produkte aus dem Stand der Technik wurden gemäß Herstellerangaben verarbeitet. Der Stand der Technik wird durch die folgenden, kommerziell erhältlichen kunststoffmodifizierten Glasionomerzemente der Firmen GC und 3M Espe representiert:
**1** Fuji II LC Improved ®, Pulver LOT 0709131, Flüssigkeit LOT 0709061,
**2** Photac Fil Quick Applicap ®, LOT 348649,

| Prüfpunkt | Stand der Technik | |
|---|---|---|
| | **1** | **2** |
| DHT [mm] | 1,9 | 2,3 |
| DH [MPa] | 170,1 | 123,8 |
| BF [MPa] | 73,5 | 37,3 |
| Löslichkeit [%] | 0,20 | 0,28 |
| Abrasion [µm] | 108,8 | 110,7 |

Übersicht der Ergebnisse Testung Anwendungsbeispiele:

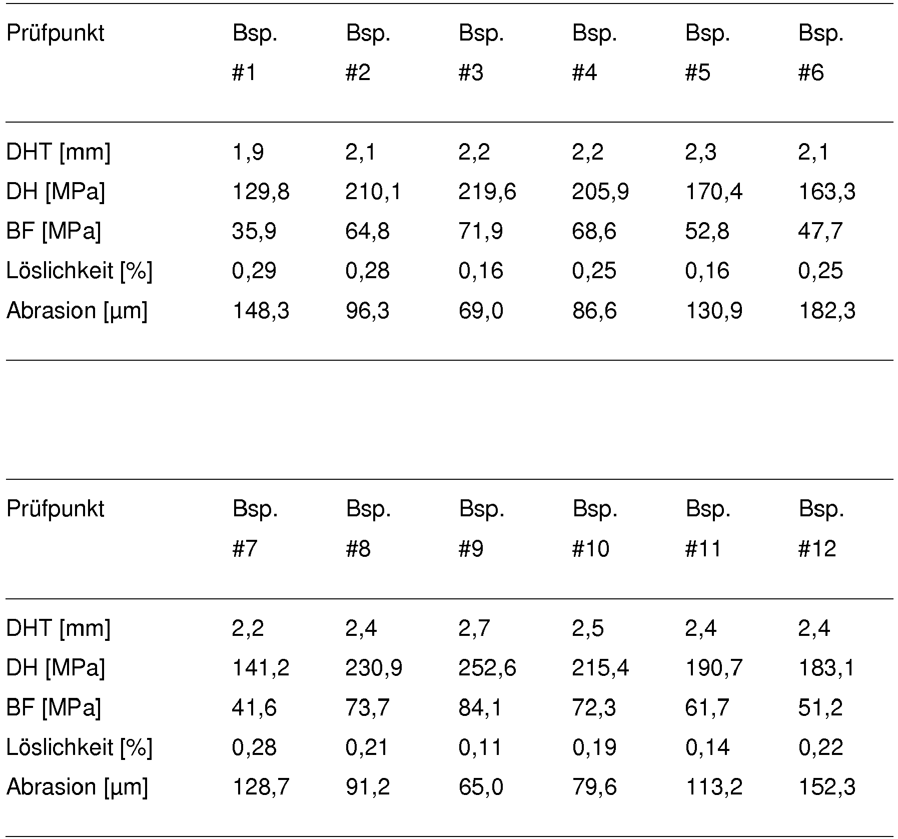

Die Versuchsergebnisse zeigen, daß die Formulierungen der erfindungsgemäßen Beispiele 3 und 4 in der oberen Tabelle die besten Werte ergeben. Bei nahezu gleichem Niveau der Druckhärte verglichen mit der Formulierung des Beispiels 2, das eigentlich die besten mechanischen Werte ergeben sollte und in dem ausschließlich feinteiliges reaktives Glas benutzt wurde, sind die Werte der Biegefestigkeit und der Abrasion der erfindungsgemäßen Zusammensetzungen überraschenderweise besser. Ändert man das Verhältnis der reaktiven Glasfraktionen zugunsten des grobteiligen Anteils (Beispiel 5), so fallen die mechanischen Werte deutlich ab. Die mit den erfindungsgemäßen Zusammensetzungen erzielten Werte sind auch gegenüber den Resultaten, die mit Zusammensetzungen aus dem Stand der Technik, wie **1** und **2** erzielt wurden, deutlich verbessert. In den Beispielen 9 und 10 der unteren Tabelle finden wir ein ähnliches Bild. Möglicherweise bedingt durch die Anwesenheit des Tricyclodecanderivats sind die mechanischen Eigenschaften hier auf höherem Niveau. Die Transluzenz der Proben 7 bis 12 ist deutlich ausgeprägter als bei den Proben 1 bis 6.

### II. System Pulver/Flüssigkeit, chemisch härtend, Hauptindikation: Befestigung, handangemischt

Die verwendete Flüssigkeit ist für alle Pulvervarianten gleich und wird nicht variiert.

**Flüssigkeit**

| Bezeichnung | Anteil [%] |
|---|---|
| BHT | 0,05 |
| UDMA | 5,00 |
| GlyDMA | 10,00 |
| HEMA | 29,95 |
| Polycarbonsäure | 25,00 |
| Wasser | 30,00 |
| Gesamt | 100,00 |

| | |
|---|---|
| BHT 3,5-Di-tert.butyl-4-hydroxytoluol UDMA Urethandimethacrylsäureester GlyDMA Glycerin-1,3-dimethacrylsäureester HEMA Hydroxyethylmethacrylsäureester Polycarbonsäure polymerisierte Acrylsäure | |

**Beispiel 1 - Pulver aus Füllstoffkombination aus reaktivem Füllstoff mit Partikelgrößenverteilung d₅₀ = 4 µm, d₉₉ = 10 µm und reaktivem Füllstoff mit Partikelgrößenverteilung d₅₀ = 8 µm, d₉₉ = 20 µm**

| Bezeichnung | Anteil [%] |
|---|---|
| BTBACI | 0,025 |
| Cu(acac)2 | 0,025 |
| Kat. | 0,300 |
| WS | 2,500 |
| Peroxid | 0,300 |
| Polycarbonsäure | 5,000 |
| reaktives Glas II | 44,150 |
| reaktives Glas I | 47,700 |
| Gesamt | 100,000 |

| | |
|---|---|
| BTBACI Benzyltributylammoniumchlorid Cu(acac)2 Kupfer(II)-acetylacetonat Kat. 1-Benzyl-5-phenylbarbitursäure Polycarbonsäure polymerisierte Acrylsäure reaktives Glas II Partikelgrößenverteilung d₅₀ = 8 µm, d₉₉ = 20 µm reaktives Glas I Partikelgrößenverteilung d₅₀ = 4 µm, d₉₉ = 10 µm WS Weinsäure Peroxid Natriumperoxodisulfat | |

**Beispiel 2 - Pulver aus Füllstoffkombination aus reaktivem Füllstoff mit Partikelgrößenverteilung d₅₀ = 4 µm, d₉₉ - 10 µm und reaktivem Füllstoff mit Partikelgrößenverteilung d₅₀ = 8 µm, d₉₉ = 20 µm**

| Bezeichnung | Anteil [%] |
|---|---|
| BTBACI | 0,025 |
| Cu(acac)2 | 0,025 |
| Kat. | 0,300 |
| WS | 2,500 |
| Peroxid | 0,300 |
| Polycarbonsäure | 5,000 |
| reaktives Glas II | 20,000 |
| reaktives Glas I | 71,850 |
| Gesamt | 100,000 |

| | |
|---|---|
| BTBACI Benzyltributylammoniumchlorid Cu(acac)2 Kupfer(II)-acetylacetonat Polycarbonsäure polymerisierte Acrylsäure Kat. 1-Benzyl-5-phenylbarbitursäure reaktives Glas II Partikelgrößenverteilung d₅₀ = 8 µm, d₉₉ = 20 µm reaktives Glas I Partikelgrößenverteilung d₅₀ = 4 µm, d₉₉ = 10 µm WS Weinsäure Peroxid Natriumperoxodisulfat | |

**Beispiel 3 - Pulver aus Füllstoffkombination aus reaktivem Füllstoff mit Partikelgrößenverteilung d₅₀ = 4 µm, d₉₉ - 10 µm und reaktivem Füllstoff mit Partikelgrößenverteilung d₅₀ = 8 µm, d₉₉ = 20 µm**

| Bezeichnung | Anteil [%] |
|---|---|
| BTBACI | 0,025 |
| Cu(acac)2 | 0,025 |
| Kat. | 0,300 |
| WS | 2,500 |
| Peroxid | 0,300 |
| Polycarbonsäure | 5,000 |
| reaktives Glas II | 50,000 |
| reaktives Glas I | 41,850 |
| Gesamt | 100,000 |

| | |
|---|---|
| BTBACI Benzyltributylammoniumchlorid Cu(acac)2 Kupfer(II)-acetylacetonat Kat. 1-Benzyl-5-phenylbarbitursäure Polycarbonsäure polymerisierte Acrylsäure reaktives Glas II Partikelgrößenverteilung d₅₀ = 8 µm, d₉₉ = 20 µm reaktives Glas I Partikelgrößenverteilung d₅₀ = 4 µm, d₉₉ = 10 µm WS Weinsäure Peroxid Natriumperoxodisulfat | |

Mit den Formulierungen der Beispiele 1 bis 3 wurde die Haftung an Rinderdentin bestimmt. Die Haftungstests wurden gemäß der Beschreibung in der DE 10 2006 014 772 durchgeführt.

| | | |
|---|---|---|
| Beispiel 1 (P/L) | 1,8:1,0 | 2,0:1,0 |
| Haftung [MPa] | 8,9 | 9,1 |

| | | | |
|---|---|---|---|
| Beispiel 2 (P/L) | 1,6:1,0 | 1,8:1,0 | 2,0:1,0 |
| Haftung [MPA] | 9,4 | 9,6 | 10,8 |

| | | | |
|---|---|---|---|
| Beispiel 3 (P/L) | 1,6:1,0 | 1,8:1,0 | 2,0:1,0 |
| Haftung [MPa] | 7,0 | 7,2 | 7,9 |

### III. System Paste/Paste, lichthärtend, Hauptindikation: Füllung, Anwendung aus Doppelkammerspritze im Verhältnis (P/P) = 1:1

### Beispiel 1

**Paste A**

| Bezeichnung | Anteil [%] |
|---|---|
| DABE | 1,250 |
| WS | 2,000 |
| UDMA | 2,500 |
| GlyDMA | 6,500 |
| HEMA | 23,000 |
| reaktives Glas II | 13,750 |
| reaktives Glas I | 51,000 |
| Gesamt | 100,000 |

| | |
|---|---|
| DABE Dimethylaminobenzoesäureethylester WS Weinsäure UDMA Urethandimethacrylat GlyDMA Glyzerindimethacrylat HEMA Hydroxyethylmethacrylat reaktives Glas II Partikelgrößenverteilung d₅₀ = 8 µm, d₉₉ = 20 µm reaktives Glas I Partikelgrößenverteilung d₅₀ = 4 µm, d₉₉ = 10 µm | |

**Paste B**

| Bezeichnung | Anteil [%] |
|---|---|
| Campherchinon | 0,750 |
| Aerosil 200 | 5,000 |
| Cristobalitmehl | 29,250 |
| Polycarbonsäure | 25,000 |
| Wasser | 40,000 |
| Gesamt | 100,000 |

| | |
|---|---|
| Polycarbonsäure polymerisierte Acrylsäure | |

Das Cristobalitmehl ist ein silanisierter inerter Füllstoff mit einer oberen Korngröße von 10 µm und einer mittleren Korngröße von 3 µm

**Ergebnisse:**

| | |
|---|---|
| DH [MPa] | 176,8 |
| BF [Mpa] | 58,9 |
| Abrasion [µm] | 71,2 |

### Beispiel 2

**Paste A**

| Bezeichnung | Anteil [%] |
|---|---|
| DABE | 1,250 |
| WS | 2,000 |
| UDMA | 2,500 |
| GlyDMA | 6,500 |
| HEMA | 23,000 |
| reaktives Glas II | 29,250 |
| reaktives Glas I | 35,500 |
| Gesamt | 100,000 |

| | |
|---|---|
| DABE Dimethylaminobenzoesäureethylester WS Weinsäure UDMA Urethandimethacrylat GlyDMA Glyzerindimethacrylat HEMA Hydroxyethylmethacrylat reaktives Glas II Partikelgrößenverteilung d₅₀ = 8 µm, d₉₉ = 20 µm reaktives Glas I Partikelgrößenverteilung d₅₀ = 4 µm, d₉₉ = 10 µm | |

**Paste B**

| Bezeichnung | Anteil [%] |
|---|---|
| Campherchinon | 0,750 |
| Aerosil 200 | 5,000 |
| Cristobalitmehl | 29,250 |
| Polycarbonsäure | 25,000 |
| Wasser | 40,000 |
| Gesamt | 100,000 |

| | |
|---|---|
| Polycarbonsäure polymerisierte Acrylsäure | |

Das Cristobalitmehl ist ein silanisierter inerter Füllstoff mit einer oberen Korngröße von 10 µm und einer mittleren Korngröße von 3 µm

**Ergebnisse:**

| | |
|---|---|
| DH4x6 [MPa] | 170,2 |
| BF2x2 [Mpa] | 53,7 |
| Abrasion [µm] | 82,3 |

### Beispiel 3

**Paste A**

| Bezeichnung | Anteil [%] |
|---|---|
| DABE | 1,250 |
| WS | 2,000 |
| UDMA | 2,500 |
| GlyDMA | 6,500 |
| HEMA | 23,000 |
| reaktives Glas II | 43,750 |
| reaktives Glas I | 21,000 |
| Gesamt | 100,000 |

| | |
|---|---|
| DABE Dimethylaminobenzoesäureethylester WS Weinsäure UDMA Urethandimethacrylat GlyDMA Glyzerindimethacrylat HEMA Hydroxyethylmethacrylat reaktives Glas II Partikelgrößenverteilung d₅₀ = 8 µm, d₉₉ = 20 µm reaktives Glas I Partikelgrößenverteilung d₅₀ = 4 µm, d₉₉ = 10 µm | |

**Paste B**

| Bezeichnung | Anteil [%] |
|---|---|
| Campherchinon | 0,750 |
| Aerosil 200 | 5,000 |
| Cristobalitmehl | 29,250 |
| Polycarbonsäure | 25,000 |
| Wasser | 40,000 |
| Gesamt | 100,000 |

| | |
|---|---|
| Polycarbonsäure polymerisierte Acrylsäure | |

Das Cristobalitmehl ist ein silanisierter inerter Füllstoff mit einer oberen Korngröße von 10 µm und einer mittleren Korngröße von 3 µm

**Ergebnisse:**

| | |
|---|---|
| DH4x6 [MPa] | 141,3 |
| BF2x2 [Mpa] | 44,5 |
| Abrasion [µm] | 108,4 |

Die aus Doppelkammerspritzen applizierten Paste/Paste-Systeme weisen gegenüber den handangemischeten Pulver/Flüssigkeitssystemen aus I geringere Füllgrade auf. Ihre mechanischen Eigenschaften liegen daher generell etwas tiefer. Allerdings ist ihr Anfließverhalten an den Kavitätenwänden besser.

### IV. System Paste/Paste, chemisch härtend, Hauptindikation: Befestigung, Wurzelfüllung, Anwendung aus Doppelkammerspritze im Verhältnis P/P 1:1

### Beispiel 1

**Paste A**

| Bezeichnung | Anteil [%] |
|---|---|
| BPO | 1,000 |
| WS | 2,500 |
| Aerosil 200 | 2,500 |
| Aerosil 8200 | 2,500 |
| UDMA | 5,000 |
| GlyDMA | 12,000 |
| HEMA | 20,000 |
| reaktives Glas II | 11,500 |
| reaktives Glas I | 43,000 |
| Gesamt | 100,000 |

| | |
|---|---|
| WS Weinsäure BPO Benzoylperoxid UDMA Urethandimethacrylat GlyDMA Glyzerindimethacrylat HEMA Hydroxyethylmethacrylat reaktives Glas II Partikelgrößenverteilung d₅₀ = 8 µm, d₉₉ = 20 µm reaktives Glas I Partikelgrößenverteilung d₅₀ = 4 µm, d₉₉ = 10 µm | |

**Paste B**

| Bezeichnung | Anteil [%] |
|---|---|
| DHEPT | 1,500 |
| Aerosil 200 | 2,500 |
| Cristobalitmehl | 18,500 |
| Polycarbonsäure | 37,500 |
| Wasser | 40,000 |
| Gesamt | 100,000 |

| | |
|---|---|
| Polycarbonsäure polymerisierte Acrylsäure DHEPT N,N-Bis-(2-hydroxyethyl)-para-toluidin | |

Das Cristobalitmehl ist ein silanisierter inerter Füllstoff mit einer oberen Korngröße von 10 µm und einer mittleren Korngröße von 3 µm

### Ergebnisse:

Haftung: 9,2 MPa

### Beispiel 2

**Paste A**

| Bezeichnung | Anteil [%] |
|---|---|
| BPO | 1,000 |
| WS | 2,500 |
| Aerosil 200 | 2,500 |
| Aerosil 8200 | 2,500 |
| UDMA | 5,000 |
| GlyDMA | 12,000 |
| HEMA | 20,000 |
| reaktives Glas II | 24,500 |
| reaktives Glas I | 30,000 |
| Gesamt | 100,000 |

| | |
|---|---|
| WS Weinsäure BPO Benzoylperoxid UDMA Urethandimethacrylat GlyDMA Glyzerindimethacrylat HEMA Hydroxyethylmethacrylat reaktives Glas II Partikelgrößenverteilung d₅₀ = 8 µm, d₉₉ = 20 µm reaktives Glas I Partikelgrößenverteilung d₅₀ = 4 µm, d₉₉ = 10 µm | |

**Paste B**

| Bezeichnung | Anteil [%] |
|---|---|
| DHEPT | 1,500 |
| Aerosil 200 | 2,500 |
| Cristobalitmehl | 18,500 |
| Polycarbonsäure | 37,500 |
| Wasser | 40,000 |
| Gesamt | 100,000 |

| | |
|---|---|
| Polycarbonsäure polymerisierte Acrylsäure DHEPT N,N-Bis-(2-hydroxyethyl)-para-toluidin | |

Das Cristobalitmehl ist ein silanisierter inerter Füllstoff mit einer oberen Korngröße von 10 µm und einer mittleren Korngröße von 3 µm

### Ergebnisse:

Haftung: 8,2 MPa

### Beispiel 3

**Paste A**

| Bezeichnung | Anteil [%] |
|---|---|
| BPO | 1,000 |
| WS | 2,500 |
| Aerosil 200 | 2,500 |
| Aerosil 8200 | 2,500 |
| UDMA | 5,000 |
| GlyDMA | 12,000 |
| HEMA | 20,000 |
| reaktives Glas II | 40,000 |
| reaktives Glas I | 24,500 |
| Gesamt | 100,000 |

| | |
|---|---|
| WS Weinsäure BPO Benzoylperoxid UDMA Urethandimethacrylat GlyDMA Glyzerindimethacrylat HEMA Hydroxyethylmethacrylat reaktives Glas II Partikelgrößenverteilung d₅₀ = 8 µm, d₉₉ = 20 µm reaktives Glas I Partikelgrößenverteilung d₅₀ = 4 µm, d₉₉ = 10 µm | |

**Paste B**

| Bezeichnung | Anteil [%] |
|---|---|
| DHEPT | 1,500 |
| Aerosil 200 | 2,500 |
| Cristobalitmehl | 18,500 |
| Polycarbonsäure | 37,500 |
| Wasser | 40,000 |
| Gesamt | 100,000 |

| | |
|---|---|
| Polycarbonsäure polymerisierte Acrylsäure DHEPT N,N-Bis-(2-hydroxyethyl)-para-toluidin | |

Das Cristobalitmehl ist ein silanisierter inerter Füllstoff mit einer oberen Korngröße von 10 µm und einer mittleren Korngröße von 3 µm

### Ergebnisse:

Haftung: 6,4 MPa

## Patentansprüche

1. Kunststoffmodifizierter Glasionomerzement enthaltend
a.) wenigstens ein Polymer einer alpha, beta - ungesättigten Carbonsäure
b.) eine basische Glaszusammensetzung, die in der Lage ist, mit wenigstens einem Polymer einer alpha, beta - ungesättigten Carbonsäure zu reagieren
c.) wenigstens ein radikalisch polymerisierbares Monomer
d.) Wasser
e.) wenigstens einen Polymerisationsinitiator
f.) optional herkömmliche Additive wie Farbmittel, Weinsäure, Kieselsäure, gegenüber dem Polymer einer alpha, beta - ungesättigten Carbonsäure inerte Füllstoffe, Stabilisatoren, Inhibitoren, Modifikatoren und bakterizid wirkende Substanzen,
**dadurch gekennzeichnet, daß** die basische Glaszusammensetzung eine Kombination aus einem ersten reaktiven Glaspulver mit einem d₅₀ Wert von 1,5 µm bis 6 µm und einem d₉₉ Wert von 7 µm bis 20 µm und einem zweiten reaktiven Glaspulver mit einem d₅₀ Wert von 7 µm bis 20 µm und einem d₉₉ Wert von 10 µm bis 25 µm darstellt, wobei der Anteil an dem ersten reaktiven Glaspulver den Anteil an dem zweiten reaktiven Glaspulver übersteigt.

2. Kunststoffmodifizierter Glasionomerzement nach Anspruch 1, **dadurch gekennzeichnet, daß** die basische Glaszusammensetzung eine Kombination aus einem ersten reaktiven Glaspulver mit einem d₅₀ Wert von 4 µm und einem d₉₉ Wert von 10 µm und einem zweiten reaktiven Glaspulver mit einem d₅₀ Wert von 8 µm und einem d₉₉ Wert von 20 µm darstellt, wobei der Anteil an dem ersten reaktiven Glaspulver den Anteil an dem zweiten reaktiven Glaspulver übersteigt.

3. Kunststoffmodifizierter Glasionomerzement nach Anspruch 1 oder 2, **dadurch gekennzeichnet, daß** er zweikomponentig in der Form Pulver/Flüssigkeit, Pulver/Paste, Flüssigkeit/Paste oder Paste/Paste vorliegt.

4. Kunststoffmodifizierter Glasionomerzement nach einem der vorstehenden Ansprüche, **dadurch gekennzeichnet, daß** der Polymerisationsinitiator ein Initiator zur Lichthärtung ist.

5. Kunststoffmodifizierter Glasionomerzement nach Anspruch 4, **dadurch gekennzeichnet, daß** der Initiator zur Lichthärtung aus einer Kombination der Katalysatoren Campherchinon/Amin und Phosphinoxid besteht.

6. Kunststoffmodifizierter Glasionomerzement nach Anspruch 1, 2 oder 3 **dadurch gekennzeichnet, daß** der Polymerisationsinitiator ein Initiator zur chemischen Härtung ist.

7. Kunststoffmodifizierter Glasionomerzement nach Anspruch 6, **dadurch gekennzeichnet, daß** der Initiator zur chemischen Härtung enthält:
a.) eine Barbitursäure oder Thiobarbitursäure, bzw. ein Barbitursäure- oder Thiobarbitursäurederivat
b.) eine Peroxodisulfat und/oder Peroxodiphosphatverbindung
c.) eine Kupferverbindung
d.) eine Verbindung mit einem ionogen vorliegendem Halogenatom

8. Kunststoffmodifizierter Glasionomerzement nach Anspruch 7, **dadurch gekennzeichnet, daß** der Initiator zur chemischen Härtung enthält:
a.) 1-Benzyl-5-phenylbarbitursäure
b.) Natriumperoxodisulfat
c.) Kupferacetylacetonat und
d.) Benzyltributylammoniumchlorid

9. Kunststoffmodifizierter Glasionomerzement nach Anspruch 1, 2 oder 3 **dadurch gekennzeichnet, daß** der Polymerisationsinitiator sowohl einen Initiator zur Lichthärtung als auch einen Initiator zur chemischen Härtung enthält.

10. Kunststoffmodifizierter Glasionomerzement nach einem der vorstehenden Ansprüche, **dadurch gekennzeichnet, daß** das Polymer der alpha, beta - ungesättigten Carbonsäure ein mittleres Molekulargewicht zwischen 40.000 und 100.000 g/mol aufweist.

11. Kunststoffmodifizierter Glasionomerzement nach einem der vorstehenden Ansprüche, **dadurch gekennzeichnet, daß** die basische Glaszusammensetzung, die in der Lage ist, mit wenigstens einem Polymer einer alpha, beta - ungesättigten Carbonsäure zu reagieren, ein Aluminiumfluorsilikatglas ist.

12. Kunststoffmodifizierter Glasionomerzement nach einem der vorstehenden Ansprüche, **dadurch gekennzeichnet, daß** die basische Glaszusammensetzung, die in der Lage ist, mit wenigstens einem Polymer einer alpha, beta - ungesättigten Carbonsäure zu reagieren, organisch oberflächenmodifiziert ist.

13. Kunststoffmodifizierter Glasionomerzement nach Anspruch 12, **dadurch gekennzeichnet, daß** die organische Oberflächenmodifizierung der basischen Glaszusammensetzung eine Silanisierung ist.

14. Kunststoffmodifizierter Glasionomerzement nach Anspruch 13, **dadurch gekennzeichnet, daß** das Silanisierungsreagens Methacryloxypropyltrialkoxysilan ist.

15. Kunststoffmodifizierter Glasionomerzement nach einem der vorstehenden Ansprüche, **dadurch gekennzeichnet, daß** die radikalisch polymerisierbaren Monomere umfassen
- wenigstens ein mindestens zwei ethylenische Gruppen aufweisendes Monomer
- wenigstens eine mindestens eine ethylenische Gruppe aufweisende Hydroxylverbindung.

16. Kunststoffmodifizierter Glasionomerzement nach Anspruch 15, **dadurch gekennzeichnet, daß** die radikalisch polymerisierbaren Monomere Hydroxyethylmethacrylat und/oder Glyzerindimethacrylat und/oder Urethandimethacrylat und/oder Dimethacrylate bzw. Diacrylate des Dihydroxymethyltricyclo[5.2.1.0^{2,6}]decans sind.

17. Kunststoffmodifizierter Glasionomerzement nach einem der vorstehenden Ansprüche, **dadurch gekennzeichnet, daß** der Zement zusätzlich zur basischen Glaszusammensetzung, die in der Lage ist, mit wenigstens einem Polymer einer alpha, beta - ungesättigten Carbonsäure zu reagieren, weiteren Füllstoff, der nicht in der Lage ist mit wenigstens einem Polymer einer alpha, beta - ungesättigten Carbonsäure zu reagieren, enthält.

18. Kunststoffmodifizierter Glasionomerzement nach Anspruch 17, **dadurch gekennzeichnet, daß** der zusätzliche Füllstoff ein Bariumaluminiumborsilikatglas und/oder Quarz und/oder Feldspat und/oder ultrafeine Kieselsäure und/oder Titandioxid und/oder Bariumsulfat und/oder eine nanoskalige, nicht aggregierte, nach dem Sol-Gel Verfahren hergestellte Kieselsäure und/oder pyrogene Kieselsäure und/oder ein durch Sprühtrocknung von Solen erhaltener aggregierter Füllstoff ist.

19. Kunststoffmodifizierter Glasionomerzement nach Anspruch 18, **dadurch gekennzeichnet, daß** der zusätzliche Füllstoff organisch oberflächenmodifiziert ist.

20. Kunststoffmodifizierter Glasionomerzement nach Anspruch 19, **dadurch gekennzeichnet, daß** die organische Oberflächenmodifizierung des zusätzlichen Füllstoffs eine Silanisierung ist.

21. Kunststoffmodifizierter Glasionomerzement nach Anspruch 20, **dadurch gekennzeichnet, daß** das Silanisierungsreagens Methacryloxypropyltrialkoxysilan ist.

22. Kunststoffmodifizierter Glasionomerzement nach einem der vorstehenden Ansprüche, **dadurch gekennzeichnet, daß** der Zement Weinsäure enthält.

23. Kunststoffmodifizierter Glasionomerzement nach einem der vorstehenden Ansprüche, **dadurch gekennzeichnet, daß** der Zement einen Inhibitor oder ein Gemisch mehrerer Inhibitoren enthält.

24. Kunststoffmodifizierter Glasionomerzement nach Anspruch 23, **dadurch gekennzeichnet, daß** der Inhibitor oder das Gemisch mehrerer Inhibitoren ausgewählt ist aus der Gruppe, die 2,6 Di-tert.-butyl-4-methylphenol, Hydrochinonmonomethylether, das 2,2 - Diphenyl - 1 - picrylhydrazylradikal, das Galvinoxylradikal, das Triphenylmethylradikal, das 2,2,6,6 - Tetramethylpiperidinyl - 1 - oxylradikal (TEMPO) oder Derivate von TEMPO, Phenothiazin oder Derivate von Phenothiazin, umfaßt.

25. Verfahren zum Herstellen eines gehärteten, kunststoffmodifizierten Glasionomerzements, der einem oder mehreren vorstehenden Ansprüchen genügt, umfassend die Schritte:
- Mischen der Komponenten gemäss Anspruch 1,
- Formen des resultierenden Gemisches zu seiner gewünschten Form,
- gegebenenfalls Bestrahlen der Mischung mit Energie einer zur Polymerisation der Monomeren geeigneten Wellenlänge.

26. Ausgehärteter dentaler Werkstoff, herstellbar durch Mischen der Komponenten eines kunststoffmodifizierten Glasionomerzements nach einem der Ansprüche 6 bis 9.

27. Ausgehärteter dentaler Werkstoff, herstellbar durch Mischen der Komponenten eines kunststoffmodifizierten Glasionomerzements nach einem der Ansprüche 4, 5 oder 9 und Bestrahlen der Mischung mit Energie einer zur Polymerisation der Monomeren geeigneten Wellenlänge.

28. Kit, enthaltend einen kunststoffmodifizierten Glasionomerzement nach Anspruch 3, wobei die Zementkomponenten vordosiert entweder in einer Misch- und Applikationskapsel oder in einer Doppelkammerspritze aufbewahrt werden.

## Claims

1. Polymer-modified glass ionomer cement containing
a.) at least one polymer of an alpha,beta-unsaturated carboxylic acid
b.) a basic glass composition which is able to react with at least one polymer of an alpha,beta-unsaturated carboxylic acid
c.) at least one free-radically polymerizable monomer
d.) water
e.) at least one polymerization initiator
f.) optionally conventional additives such as colorants, tartaric acid, silica, fillers which are inert towards the polymer of an alpha,beta-unsaturated carboxylic acid, stabilizers, inhibitors, modifiers and bacteriocidal substances,
**characterized in that** the basic glass composition is a combination of a first reactive glass powder having a d₅₀ of from 1.5 µm to 6 µm and a d₉₉ of from 7 µm to 20 µm and a second reactive glass powder having a d₅₀ of from 7 µm to 20 µm and a d₉₉ of from 10 µm to 25 µm, where the proportion of the first reactive glass powder is greater than the proportion of the second reactive glass powder.

2. Polymer-modified glass ionomer cement according to Claim 1, **characterized in that** the basic glass composition is a combination of a first reactive glass powder having a d₅₀ of from 4 µm and a d₉₉ of 10 µm and a second reactive glass powder having a d₅₀ of 8 µm and a d₉₉ of 20 µm, where the proportion of the first reactive glass powder is greater than the proportion of the second reactive glass powder.

3. Polymer-modified glass ionomer cement according to Claim 1 or 2, **characterized in that** it is present in two-component form as powder/liquid, powder/paste, liquid/paste or paste/paste.

4. Polymer-modified glass ionomer cement according to any of the preceding claims, **characterized in that** the polymerization initiator is an initiator for light curing.

5. Polymer-modified glass ionomer cement according to Claim 4, **characterized in that** the initiator for light curing consists of a combination of the catalysts camphorquinone/amine and phosphine oxide.

6. Polymer-modified glass ionomer cement according to Claim 1, 2 or 3, **characterized in that** the polymerization initiator is an initiator for chemical curing.

7. Polymer-modified glass ionomer cement according to Claim 6, **characterized in that** the initiator for chemical curing contains:
a.) a barbituric acid or thiobarbituric acid, or a barbituric acid derivative or thiobarbituric acid derivative
b.) a peroxodisulphate and/or peroxodiphosphate compound
c.) a copper compound
d.) a compound having a halogen atom present in ionogenic form.

8. Polymer-modified glass ionomer cement according to Claim 7, **characterized in that** the initiator for chemical curing contains:
a.) 1-benzyl-5-phenyl barbituric acid
b.) sodium peroxodisulphate
c.) copper acetylacetonate and
d.) benzyltributylammonium chloride.

9. Polymer-modified glass ionomer cement according to Claim 1, 2 or 3, **characterized in that** the polymerization initiator contains both an initiator for light curing and an initiator for chemical curing.

10. Polymer-modified glass ionomer cement according to any of the preceding claims, **characterized in that** the polymer of the alpha,beta-unsaturated carboxylic acid has an average molecular weight of from 40 000 to 100 000 g/mol.

11. Polymer-modified glass ionomer cement according to any of the preceding claims, **characterized in that** the basic glass composition which is able to react with at least one polymer of an alpha,beta-unsaturated carboxylic acid is an aluminium fluorosilicate glass.

12. Polymer-modified glass ionomer cement according to any of the preceding claims, **characterized in that** the basic glass composition which is able to react with at least one polymer of an alpha,beta-unsaturated carboxylic acid is organically surface-modified.

13. Polymer-modified glass ionomer cement according to Claim 12, **characterized in that** the organic surface modification of the basic glass composition is a silanization.

14. Polymer-modified glass ionomer cement according to Claim 13, **characterized in that** the silanizing reagent is methacryloxypropyltrialkoxysilane.

15. Polymer-modified glass ionomer cement according to any of the preceding claims, **characterized in that** the free-radically polymerizable monomers comprise
- at least one monomer having at least two ethylenic groups
- at least one hydroxyl compound having at least one ethylenic group.

16. Polymer-modified glass ionomer cement according to Claim 15, **characterized in that** the free-radically polymerizable monomers are hydroxyethyl methacrylate and/or glyceryl dimethacrylate and/or urethane dimethacrylate and/or dimethacrylate or diacrylates of dihydroxymethyltricyclo[5.2.1.0^{2,6}]-decane.

17. Polymer-modified glass ionomer cement according to any of the preceding claims, **characterized in that** the cement contains a further filler which is not able to react with at least one polymer of an alpha,beta-unsaturated carboxylic acid in addition to the basic glass composition which is able to react with at least one polymer of an alpha,beta-unsaturated carboxylic acid.

18. Polymer-modified glass ionomer cement according to Claim 17, **characterized in that** the additional filler is a barium aluminium borosilicate glass and/or quartz and/or feldspar and/or ultrafine silica and/or titanium dioxide and/or barium sulphate and/or a nanosize, unaggregated silica produced by the sol-gel process and/or pyrogenic silica and/or an aggregated filler obtained by spray drying of sols.

19. Polymer-modified glass ionomer cement according to Claim 18, **characterized in that** the additional filler is organically surface-modified.

20. Polymer-modified glass ionomer cement according to Claim 19, **characterized in that** the organic surface modification of the additional filler is a silanization.

21. Polymer-modified glass ionomer cement according to Claim 20, **characterized in that** the silanizing reagent is methacryloxypropyltrialkoxysilane.

22. Polymer-modified glass ionomer cement according to any of the preceding claims, **characterized in that** the cement contains tartaric acid.

23. Polymer-modified glass ionomer cement according to any of the preceding claims, **characterized in that** the cement contains an inhibitor or a mixture of a plurality of inhibitors.

24. Polymer-modified glass ionomer cement according to Claim 23, **characterized in that** the inhibitor or the mixture of a plurality of inhibitors is selected from the group consisting of 2,6-di-tert-butyl-4-methylphenol, hydroquinone monomethyl ether, the 2,2-diphenyl-1-picrylhydrazyl radical, the galvinoxyl radical, the triphenylmethyl radical, the 2,2,6,6-tetramethylpiperidinyl-1-oxyl radical (TEMPO) or derivatives of TEMPO, phenothiazine or derivatives of phenothiazine.

25. Process for producing a cured, polymer-modified glass ionomer cement according to one or more of the above claims, which comprises the steps:
- mixing of the components according to Claim 1,
- shaping of the resulting mixture to its desired shape,
- optionally irradiation of the mixture with energy having a wavelength suitable for polymerization of the monomers.

26. Cured dental material producible by mixing of the components of a polymer-modified glass ionomer cement according to any of Claims 6 to 9.

27. Cured dental material producible by mixing of the components of a polymer-modified glass ionomer cement according to any of Claims 4, 5 or 9 and irradiation of the mixture with energy having a wavelength suitable for polymerization of the monomers.

28. Kit containing a polymer-modified glass ionomer cement according to Claim 3, wherein the cement components are stored in premetered form either in a mixing and application capsule or in a double-chamber syringe.

## Revendications

1. Ciment verre ionomère modifié à la résine, contenant :
a.) au moins un polymère d'un acide carboxylique alpha,bêta-insaturé,
b.) une composition de verre basique, qui est en mesure de réagir avec au moins un polymère d'un acide carboxylique alpha,bêta-insaturé,
c.) au moins un monomère polymérisable par voie radicalaire,
d.) de l'eau,
e.) au moins un initiateur de polymérisation,
f.) éventuellement des additifs usuels tels que des colorants, l'acide tartrique, la silice, des charges inertes vis-à-vis du polymère d'un acide carboxylique alpha,bêta-insaturé, des stabilisateurs, des inhibiteurs, des modificateurs, des substances à effet bactéricide,
**caractérisé en ce que** la composition de verre basique est une combinaison d'une première poudre de verre réactive ayant une valeur d₅₀ de 1,5 µm à 6 µm et une valeur d₉₉ de 7 µm à 20 µm, et d'une deuxième poudre de verre réactive ayant une valeur d₅₀ de 7 µm à 20 µm et une valeur d₉₉ de 10 µm à 25 µm, la proportion de la première poudre de verre réactive dépassant la proportion de la deuxième poudre de verre réactive.

2. Ciment verre ionomère modifié à la résine selon la revendication 1, **caractérisé en ce que** la composition de verre basique est une combinaison d'une première poudre de verre réactive ayant une valeur d₅₀ de 4 µm et une valeur d₉₉ de 10 µm, et d'une deuxième poudre de verre réactive ayant une valeur d₅₀ de 8 µm et une valeur d₉₉ de 20 µm, la proportion de la première poudre de verre réactive dépassant la proportion de la deuxième poudre de verre réactive.

3. Ciment verre ionomère modifié à la résine selon la revendication 1 ou 2, **caractérisé en ce qu'**il se présente sous forme bicomposante sous la forme poudre/liquide, poudre/pâte, liquide/pâte ou pâte/pâte.

4. Ciment verre ionomère modifié à la résine selon l'une quelconque des revendications précédentes, **caractérisé en ce que** l'initiateur de polymérisation est un initiateur pour le durcissement à la lumière.

5. Ciment verre ionomère modifié à la résine selon la revendication 4, **caractérisé en ce que** l'initiateur pour le durcissement à la lumière est constitué par une combinaison des catalyseurs camphre-quinone/amine et phosphinoxyde.

6. Ciment verre ionomère modifié à la résine selon la revendication 1, 2 ou 3, **caractérisé en ce que** l'initiateur de polymérisation est un initiateur pour le durcissement chimique.

7. Ciment verre ionomère modifié à la résine selon la revendication 6, **caractérisé en ce que** l'initiateur pour le durcissement chimique contient :
a.) un acide barbiturique ou un acide thiobarbiturique ou un dérivé d'acide barbiturique ou d'acide thiobarbiturique,
b.) un peroxodisulfate et/ou un composé de peroxodiphosphate,
c.) un composé de cuivre,
d.) un composé contenant un atome d'halogène présent sous forme ionogène.

8. Ciment verre ionomère modifié à la résine selon la revendication 7, **caractérisé en ce que** l'initiateur pour le durcissement chimique contient :
a.) de l'acide 1-benzyl-5-phénylbarbiturique,
b.) du peroxodisulfate de sodium,
c.) de l'acétylacétonate de cuivre, et
d.) du chlorure de benzyltributylammonium.

9. Ciment verre ionomère modifié à la résine selon la revendication 1, 2 ou 3, **caractérisé en ce que** l'initiateur de polymérisation contient aussi bien un initiateur pour le durcissement à la lumière qu'un initiateur pour le durcissement chimique.

10. Ciment verre ionomère modifié à la résine selon l'une quelconque des revendications précédentes, **caractérisé en ce que** le polymère de l'acide carboxylique alpha,bêta-insaturé présente un poids moléculaire moyen compris entre 40 000 et 100 000 g/mol.

11. Ciment verre ionomère modifié à la résine selon l'une quelconque des revendications précédentes, **caractérisé en ce que** la composition de verre basique qui est en mesure de réagir avec au moins un polymère d'un acide carboxylique alpha,bêta-insaturé est un verre de fluorosilicate d'aluminium.

12. Ciment verre ionomère modifié à la résine selon l'une quelconque des revendications précédentes, **caractérisé en ce que** la composition de verre basique qui est en mesure de réagir avec au moins un polymère d'un acide carboxylique alpha,bêta-insaturé est modifiée organiquement en surface.

13. Ciment verre ionomère modifié à la résine selon la revendication 12, **caractérisé en ce que** la modification organique de surface de la composition de verre basique est une silanisation.

14. Ciment verre ionomère modifié à la résine selon la revendication 13, **caractérisé en ce que** le réactif de silanisation est le méthacryloxypropyltrialcoxysilane.

15. Ciment verre ionomère modifié à la résine selon l'une quelconque des revendications précédentes, **caractérisé en ce que** les monomères polymérisables par voie radicalaire comprennent :
- au moins un monomère comprenant au moins deux groupes éthyléniques,
- au moins un composé hydroxyle comprenant au moins un groupe éthylénique.

16. Ciment verre ionomère modifié à la résine selon la revendication 15, **caractérisé en ce que** les monomères polymérisables par voie radicalaire sont le méthacrylate d'hydroxyéthyle et/ou le diméthacrylate de glycérine et/ou le diméthacrylate d'uréthane et/ou les diméthacrylates ou diacrylates de dihydroxyméthyltricyclo[5.2.1.0^{2,6}]décane.

17. Ciment verre ionomère modifié à la résine selon l'une quelconque des revendications précédentes, **caractérisé en ce que** le ciment contient en plus de la composition de verre basique qui est en mesure de réagir avec au moins un polymère d'un acide carboxylique alpha,bêta-insaturé une autre charge qui n'est pas en mesure de réagir avec au moins un polymère d'un acide carboxylique alpha,bêta-insaturé.

18. Ciment verre ionomère modifié à la résine selon la revendication 17, **caractérisé en ce que** la charge supplémentaire est un verre de borosilicate de baryum et d'aluminium et/ou du quartz et/ou du feldspath et/ou une silice ultrafine et/ou du dioxyde de titane et/ou du sulfate de baryum et/ou une silice nanométrique non agrégée fabriquée par le procédé sol-gel et/ou une silice pyrogénée et/ou une charge agrégée obtenue par séchage par pulvérisation de saumures.

19. Ciment verre ionomère modifié à la résine selon la revendication 18, **caractérisé en ce que** la charge supplémentaire est modifiée organiquement en surface.

20. Ciment verre ionomère modifié à la résine selon la revendication 19, **caractérisé en ce que** la modification organique de surface de la charge supplémentaire est une silanisation.

21. Ciment verre ionomère modifié à la résine selon la revendication 20, **caractérisé en ce que** le réactif de silanisation est le méthacryloxypropyltrialcoxysilane.

22. Ciment verre ionomère modifié à la résine selon l'une quelconque des revendications précédentes, **caractérisé en ce que** le ciment contient de l'acide tartrique.

23. Ciment verre ionomère modifié à la résine selon l'une quelconque des revendications précédentes, **caractérisé en ce que** le ciment contient un inhibiteur ou un mélange de plusieurs inhibiteurs.

24. Ciment verre ionomère modifié à la résine selon la revendication 23, **caractérisé en ce que** l'inhibiteur ou le mélange de plusieurs inhibiteurs est choisi dans le groupe constitué par le 2,6-di-tert.-butyl-4-méthylphénol l'éther monométhylique d'hydroquinone, le radical 2,2-diphényl-1-picrylhydrazyle, le radical galvinoxyle, le radical triphénylméthyle, le radical 2,2,6,6-tétraméthylpipéridinyl-1-oxyle (TEMPO) ou des dérivés de TEMPO, la phénothiazine ou des dérivés de phénothiazine.

25. Procédé de fabrication d'un ciment verre ionomère modifié à la résine durci qui satisfait une ou plusieurs des revendications précédentes, comprenant les étapes suivantes :
- le mélange des composants selon la revendication 1,
- le façonnage du mélange résultant en sa forme souhaitée,
- éventuellement l'exposition du mélange à une énergie d'une longueur d'onde appropriée pour la polymérisation des monomères.

26. Matériau dentaire durci, pouvant être fabriqué par mélange des composants d'un ciment verre ionomère modifié à la résine selon l'une quelconque des revendications 6 à 9.

27. Matériau dentaire durci, pouvant être fabriqué par mélange des composants d'un ciment verre ionomère modifié à la résine selon l'une quelconque des revendications 4, 5 ou 9 et exposition du mélange à une énergie d'une longueur d'onde appropriée pour la polymérisation des monomères.

28. Kit contenant un ciment verre ionomère modifié à la résine selon la revendication 3, dans lequel les composants du ciment sont stockés pré-dosés dans une capsule de mélange et d'application ou dans une seringue à chambre double.
